(19) Europäisches Patentamt
European Patent Office
Office européen des brevets

(11) Publication number: **0 301 813 B1**

(12) # EUROPEAN PATENT SPECIFICATION

(45) Date of publication of the patent specification: **08.08.90**

(51) Int. Cl.[5]: **C07C 59/64**, C07D 215/14, C07D 235/12, C07D 213/30, C07D 277/64, C07D 277/24, A61K 31/19, A61K 31/33

(21) Application number: **88306888.4**

(22) Date of filing: **26.07.88**

(54) **Naphthalenepropionic acid derivatives.**

(30) Priority: **31.07.87 US 80122**
**10.06.88 US 202975**

(43) Date of publication of application:
**01.02.89 Bulletin 89/5**

(45) Publication of the grant of the patent:
**08.08.90 Bulletin 90/32**

(84) Designated Contracting States:
**AT BE CH DE ES FR GR IT LI LU NL SE**

(56) References cited:
**EP-A- 0 143 371**
**US-A- 4 001 301**

(73) Proprietor: **AMERICAN HOME PRODUCTS CORPORATION, 685, Third Avenue, New York, New York 10017(US)**

(72) Inventor: **Kreft, Anthony Frank, 512 Lexington Lane, Trooper Pennsylvania(US)**
Inventor: **Musser, John Henry, 1009 Millstream Drive, Malvern Pennsylvania(US)**
Inventor: **Bicksler, James Jacob, 1074 Bayless Place, Eagleville Pennsylvania(US)**
Inventor: **Giberson, John William, 3000 Valley Forge Circle Apartment 251, King of Prussia Pennsylvania(US)**
Inventor: **Kubrak, Dennis Martin, 856 Eaton Road, Drexel Hill Pennsylvania 19026(US)**

(74) Representative: **Porter, Graham Ronald et al, c/o Wyeth Laboratories Huntercombe Lane South, Taplow Maidenhead Berkshire SL6 0PH(GB)**

EP 0 301 813 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to novel naphthalenepropionic acid derivatives possessing lipoxygenase inhibitory and leukotriene antagonist activity, which are useful as anti-inflammatory, antiallergic and cytoprotective agents.

It is known that arachidonic acid (AA) is metabolized in mammals by two distinct pathways. The metabolism of arachidonic acid by cyclooxygenase enzymes results in the production of prostaglandins and thromboxanes. The physiological activity of the prostaglandins has already been amply elucidated in recent years. The other pathway of AA metabolism involves lipoxygenase enzymes and results in the production of a number of oxidative products called leukotrienes. The latter are designated by the LT nomenclature system, and the most significant products of the lipoxygenase metabolic pathway are the leukotrienes $B_4$, $C_4$, $D_4$ and $E_4$. The substance denominated slow-reacting substance of anaphylaxis (SRS-A) has been shown to consist of a mixture of sulfidopeptide leukotrienes, $C_4$, $D_4$ and $E_4$ [see Bach et al., J. Immun.215, 115-118 (1980); Biochem. Biophys. Res. Commun. 93 1121-1126 (1980)].

The significance of these leukotrienes is that a great deal of evidence has been accumulated showing that leukotrienes participate in inflammatory reactions, exhibit chemotactic activities, stimulate lysosomal enzyme release and act as important factors in the immediate hypersensitivity reaction. It has been shown that $LTC_4$ and $LTD_4$ are potent bronchoconstrictors of the human bronchi [see Dahlen et al., Nature 288, 484-486 (1980) and Piper, Int. Arch. Apple. Immunol., 76, suppl. 1, 43 (1985)] which stimulate the release of mucus from airways in vitro [Marom et al., Am. Rev. Resp. Dis., 126, 449 (1982], are potent vasodilators in skin [see Bisgaard et al, Prostaglandins, 23, 797 (1982)], and produce a wheal and flare response [Camp et al., Br. J. Pharmacol, 80, 497 (1983)]. The nonpeptide leukotriene, $LTB_4$, is a powerful chemotactic factor for leukocytes [see A.W. Ford-Hutchinson, J. Roy. Soc. Med., 74, 831-833 (1981)], which stimulates cell accumulation and affects vascular smooth muscle [see Bray, Br. Med. Bull., 39, 249 (1983)]. The activity of leukotrienes as mediators of imflammation and hypersensitivity is extensively reviewed in Bailey and Casey, Ann. Reports Med. Chem., 17, 203-217 (1982) and in Bray, Agents and Actions, 19, 87 (1986).

There is also evidence that products of the cyclooxygenase/lipoxygenase pathways play key roles in both the pathogenesis of gastric mucosal damage due to extracellular (gastric and intestinal contents, microorganisms, and the like) or intracellular (ischemia, viruses, etc.) agents, as well as in cytoprotection against such damage. Thus, on the one hand prostaglandins exert a cytoprotective effect on the gastric mucosa [see Robert., Gastroenterology, 77, 761-767 (1979)] and this action of the prostaglandins, especially of the E series, is considered to be of importance in the treatment of gastrointestinal ulceration [see Isselbacher, Drugs, 33 (Suppl.), 38-46 (1987)]. On the other hand, ex vivo experiments have shown that gastric mucosal tissue from ethanol-pretreated rats is capable of $LT_4$ generation and that this $LTC_4$ production is quantitatively related to the severity of the ethanol damage [see Lange et al., Naunyn-Schmiedeberg's Arch. Pharmacol. Suppl., 330, R27, (1985)]. It has also been demonstrated that $LTC_4$ can induce vaso-construction in both venous and arteriolar vessels in the rat submucosa [see Whittle, IUPHAR Ninth Int. Cong. of Pharm., S30-2, London, England (1984)]. This is significant since ethanol-induced lesion formation in gastric mucosa may be multifactorial with, for example, stasis of gastric blood flow contributing significantly to the development of the hemorrhagic necrotic aspects of the tissue injury [see Guth et al., Gastroenterology, 87, 1083-90 (1984)]. Moreover, in the anesthetized cat, exogenous $LTD_4$ evokes both increased pepsin secretion and decreased transgastric potential [Pendleton et al., Eur.J. Pharmacol., 125, 297-99 (1986)]. A particularly significant recent finding in this regard is that 5-lipoxygenase inhibitors and some leukotriene antagonists protect the gastric mucosa against lesions induced by the oral or parenteral administration of most nonsteroidal antiinflammatory drugs [see Rainsford, Agents and Actions, 21, 316-19 (1987)]. Accordingly, a significant body of evidence implicates the involvement of lipoxygenase products in the development of pathological features associated with gastric mucosal lesions, such as for example those induced by ethanol exposure and administration of nonsteroidal anti-inflammatory drugs. Thus compounds which inhibit the biological effects of leukotrienes and/or which control the biosynthesis of these substances, as by inhibiting 5-lipoxygenase, are considered to be of value as cytoprotective agents.

Accordingly, the biological activity of the leukotrienes and SRS's, and of lipoxygenase as the enzyme leading to the metabolism of AA to leukotrienes, indicates that a rational approach to drug therapy to prevent, remove or ameliorate the symptoms of allergies, anaphylaxis, asthma and inflammation and for gastric cytoprotection must focus on either blocking the release of mediators of these conditions or antagonizing their effects. Thus compounds, which inhibit the biological effects of the leukotrienes and SRS's and /or which control the biosynthesis of these substances, as by inhibiting lipoxygenase, are considered to be of value in treating such conditions as allergic bronchial asthma, allergic rhinitis, as well as in other immediate hypersensitivity reactions and in providing gastric cytoprotection.

US-A 4 001 301 discloses 6-substituted 2-naphthylacetic and propionic acid derivatives which have antiinflammatory, analgesic, antipyretic and anti-pruritic activity.

It has now been found that certain novel naphthalenepropionic acid derivatives inhibit lipoxygenase and antagonize products of the lipoxygenase pathway, and so are useful as anti-inflammatory, anti-allergic and cytoprotective agents. The present invention provides novel compounds having the following formula:

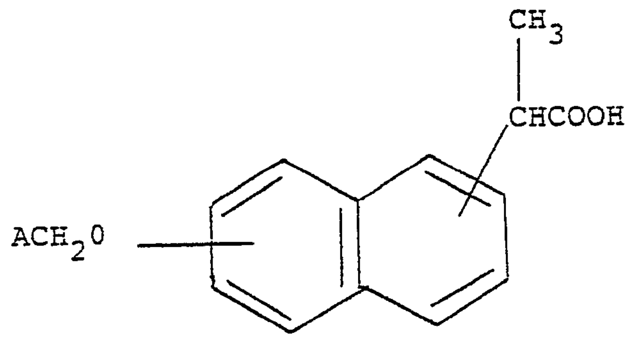

wherein A is alkyl of 3–19 carbon atoms, diloweralkylvinyl, dihalovinyl, diphenylvinyl, lower alkynyl,

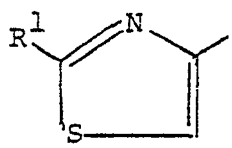

or

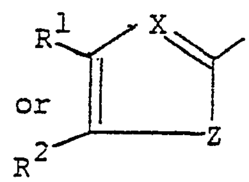

;

X is N or CR;

Z is 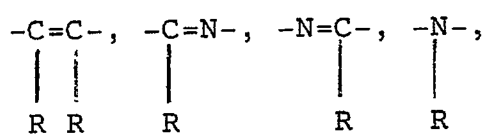 S or O;

R is hydrogen or lower alkyl;
$R^1$ is hydrogen, lower alkyl or phenyl;
$R^2$ is hydrogen or lower alkyl; or

$R^1$ and $R^2$ taken together with the carbon atoms to which they are attached form a benzene ring; and the pharmaceutically acceptable salts thereof, with the proviso that A is not 3 to 5 carbon atoms when $ACH_2O$ is in the 6-position.

The terms "lower alkyl" and lower alkynyl refer to moieties having 1 to 6 carbon atoms in the carbon chain.

The compounds may be prepared by reacting a compound of formula II

$ACH_2$ Hal

II

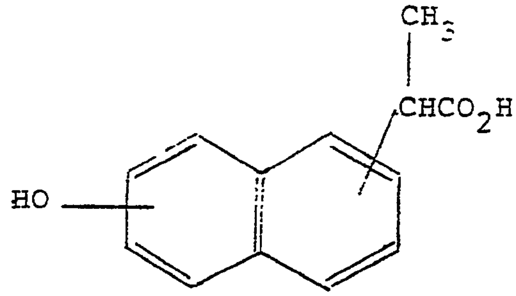

III

wherein A is as defined above and Hal is a halogen atom selected from chlorine, bromine and iodine, with one equivalent of a metal derivative, eg. a dialkali metal, eg. disodio, derivative, of a compound of formula III. Alternatively 2 equivalents the compound II may be used to give an ether ester of formula IVa eg IV

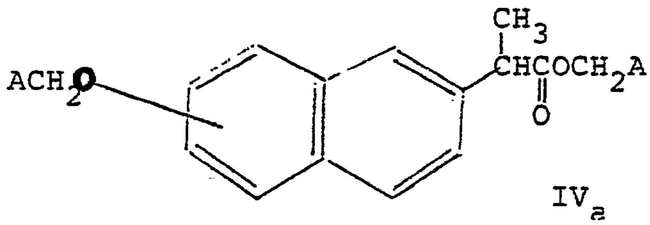

IVa

An example is a compound of formula IV

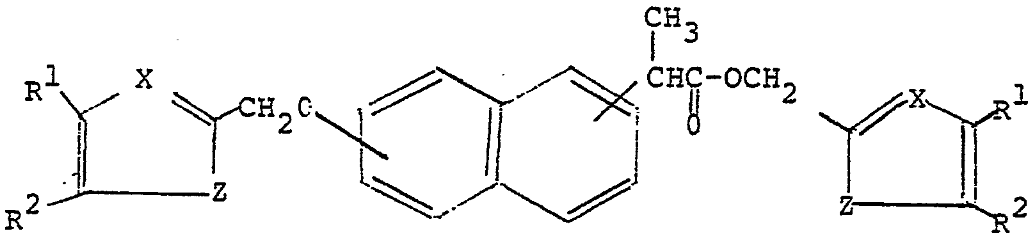

The ether ester of formula IVa is then hydrolysed eg. with dilute alkali metal hydroxide such as sodium hydroxide to obtain the compound of formula I. The metal derivative of the acid of formula III may be prepared by treating the acid of formula III with an alkali metal alkoxide such as sodium methoxide.

In a further alternative process an ester IIIa of the acid III is alkylated with compound II in the presence of a base eg. $K_2CO_3$

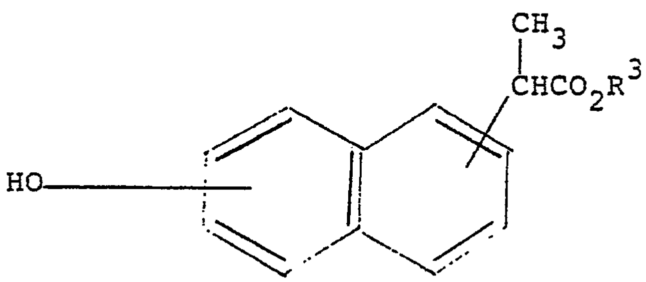

IIIa

to give an ester IVb

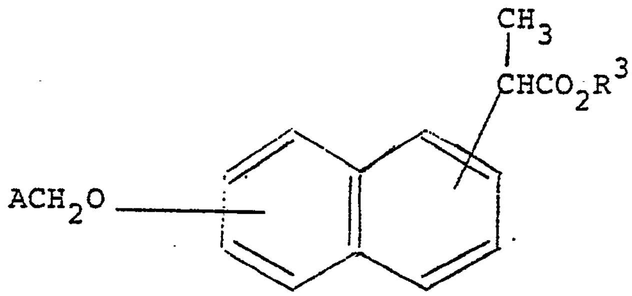

which is then hydrolysed eg with a dilute hydroxide such as for example sodium hydroxide to compound I. $R^3$ in compounds IIIa and IIIb maybe an alkyl group eg. a lower alkyl group of 1 to 6 carbon atoms preferably methyl and A is as defined above.

Examples of the radical A are 2-benzthiazolyl, 2-naphthalenyl, 2 quinolyl, 2-phenyl-4-thiazolyl, 2,2-dichlorovinyl and 2,2 - dimethylvinyl.

The starting materials of formula II, III and IIIa as used in the above reaction sequences are available commercially or can be prepared by known methods conventional in the art. Thus, for example, the benzo-fused heterocyclic compounds such as 1-methyl-2-chloromethylbenzimidazole 2-chloromethylbenzthiazole and 2-chloromethylbenzoxazole can be prepared by the following reaction scheme:

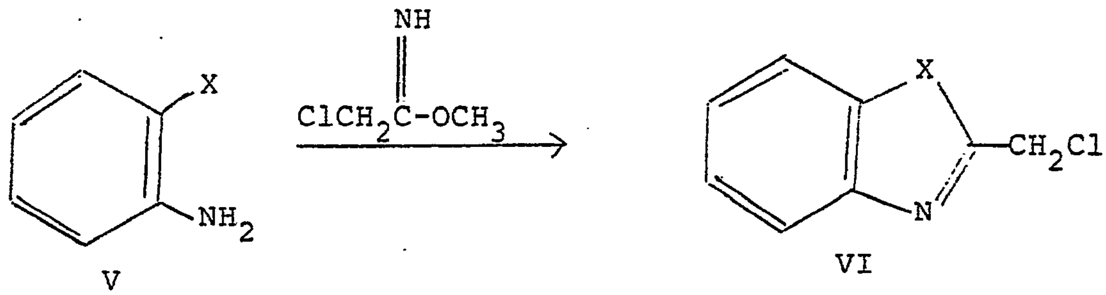


V

VI

wherein X is O, S or $NCH_3$. The reaction is preferably carried out at a controlled low temperature in an organic solvent, such as methylene chloride.

Compounds of the invention which contain a basic nitrogen are capable of forming pharmaceutically acceptable salts, including the salts of pharmaceutically acceptable organic and inorganic acids such as hydrochloric, hydrobromic, sulfuric, nitric, phosphoric, methanesulfonic, benzenesulfonic , acetic, citric, fumaric, maleic, succinic and the like. The carboxylic acid function in the compounds of formula I is capable of forming salts with pharmaceutically acceptable cations eg alkali-metal, alkaline earth metal salts and ammonium and amine salts.

The compounds of the invention, by virtue of their ability to inhibit the activity of lipoxygenase enzyme and to antagonize mediators arising from this enzymatic pathway, are useful in the treatment of inflammatory conditions. Accordingly, the compounds are indicated in the treatment of such diseases as rheumatoid arthritis, osteoarthritis, tendinitis, bursitis and similar conditions involving inflammation. Moreover, by virtue of their ability to inhibit the activity of lipoxygenase enzyme and by their ability to antagonize the effect of $LTC_4$, $LTD_4$ and $LTE_4$ which are the constituents of SRS-A, they are useful for the inhibition of symptons induced by these leukotrienes. Accordingly, the compounds are indicated in the prevention and treatment of those disease states in which $LTC_4$, $LTD_4$ and $LTE_4$ are causative factors, for example allergic rhinitis, allergic bronchial asthma and other leukotriene mediated naso-bronchial obstructive air-passageway conditions, as well as in other immediate hypersensitivity reactions, such as allergic conjunctivitis. The compounds are especially valuable in the prevention and treatment of allergic bronchial asthma.

The compounds of the invention are cytoprotective agents and are considered especially useful when administered with conventional non-steroidal anti-inflammatory drugs, whose major side effect is gastrointestinal irritation. The cytoprotective effect of the compounds of the invention significantly reduces the gastroirritant impact of conventional anti-inflammatory drugs. This effect is based not only on the ability of the compounds of the invention to inhibit the biological effects of leukotrienes and/or control the biosynthesis of these substances, as by inhibiting lipoxygenase, but also by a shunting effect, whereby the control of the lipoxygenase pathway shunts the oxidation of arachidonic acid into the cyclooxygenase pathway, giving rise to an increase in the formation of cytoprotective prostaglandins. These biological effects make the compounds of the invention especially useful in treating such conditions as erosive esophagitis, inflammatory bowel diesease and induced hemorrhagic lesions such as those induced by alcohol or non-steroidal anti-inflammatory drugs (NSAID's), hepatic ischemia, noxious agent induced damage or necrosis of hepatic, pancreatic, renal or myocardial tissue; liver parenchymal damage caused by hepatotoxic agents such as carbon tetrachloride and D-galactosamine; ischemic renal failure; disease-induced hepatic damage; bile salt-induced pancreatic or gastric damage; trauma or stress-induced cell damage; and glycerol-induced renal failure.

When the compounds of the invention are employed in the treatment of allergic airway disorders, as antiinflammatory agents, and/or as cytoprotective agents they can be formulated into oral dosage forms such as tablets, capsules and the like. The compounds can be administered alone or by combining them with conventional carriers, such as magnesium carbonate, magnesium stearate, talc, sugar, lactose, pectin, dextrin, starch, gelatin, tragacanth, methylcellulose, sodium carboxymethylcellulose, low melting wax, cocoa butter and the like. Diluents, flavouring agents, solubilizers, lubricants, suspending agents, binders, tablet-disintegrating agents and the like may be employed. The compounds may be encapsulated with or without other carriers. In all cases, the proportion of active ingredients in said compositions both solid and liquid will be at least to impart the desired activity thereto on oral administration. The compounds may also be injected parenterally, in which case they are used in the form of a sterile solution containing other solutes, for example, enough saline or glucose to make the solution isotonic. For administration by inhalation or insufflation, the compounds may be formulated into an aqueous or partially aqueous solution, which can then be utilized in the form of an aerosol.

The dosage requirements vary with the particular compositions employed, the route of administration, the severity of the symptoms presented and the particular subject being treated. Treatment will generally be initiated with small dosages less than the optimum dose of the compound. Thereafter the dosage is increased until the optimum effect under the circumstances is reached. In general, the compounds of the

invention are most desirably administered at a concentration that will generally afford effective results without causing any harmful or deleterious side effects, and can be administered either as a single unit dose, or if desired, the dosage may be divided into convenient subunits administered at suitable times throughout the day.

The lipoxygenase inhibitory and leukotriene antagonist effects as well as the antiinflammatory and cytoprotective effects of the compounds of the invention may be demonstrated by standard pharmacological procedures, which are described more fully in the examples given hereinafter.

These procedures illustrate the ability of the compounds of the invention to inhibit the polymorphonuclear leukocyte synthesis of the lipoxygenase product 5-HETE; the in vivo ability of the compounds to inhibit bronchospasm induced by exogenously administered mediators of bronnchoconstriction; and measure the in vivo activity of the compounds as antiinflammatory agents in the rat carrageenan paw edema assay; measure the potential of the compounds to induce acute gastroirritation in rats; measure the ability of the compounds to prevent acute gastroirritation in rats induced by non-steroidal anti-inflammatory drugs.

The following examples show the preparation and pharmacological testing of compounds within the invention.

Example 1

α-Methyl-6-(2-quinolinylmethoxy)-2-naphthaleneacetic acid

To a solution of 6-hydroxy-α-methyl-2-naphthaleneacetic acid (10.8g, 50mmol) in methanol (100ml) is added sodium methoxide (100mmol). After 10 minutes the solvent is removed and replaced with dimethylformamide (250ml). 2-(Chloromethyl)quinoline (17.8g, 100mmol) is then added and the reaction mixture is stirred for 8 days at room temperature. The reaction mixture is partioned between water and methylene chloride, the organic layer is washed with water and evaporated to yield 27g of an oil. Recrystallization of this oil twice from acetonitrile gives 10.6g of white crystals of intermediate ether ester (42% yield, m.p. 106-108°C).

The ether ester from above is hydrolyzed as follows: a solution of the ether ester (14.4g, 28.9 mmol) in a mixture of 110ml of 1N NaOH and 110ml tetrahydrofuran is refluxed for 1 hour. The organic solvent is then removed and 2-(hydroxymethyl)quinoline is filtered off. The aqueous solution is acidified to pH 6 and the precipitate is filtered and recrystallized from ethanol to afford 2.2g of white crystals (21% yield, m.p. 186-187°C).

Analysis for: $C_{23}H_{19}NO_3$
Calculated: C, 77.30;H, 5.36; N, 3.92.
Found: C, 77.69;H, 5.36;N, 3.93.

Example 2

α-Methyl-6-(phenylmethoxy)-2-naphthaleneactic acid

To a solution of 6-hydroxy-α-methyl-2-naphthaleneacetic acid (6.48g, 30mmol) in methanol (100ml) is added sodium methoxide (60mmol). After 10 minutes the solvent is removed and replaced with dimethylformamide (100ml). Benzyl chloride (7.6g, 60 mmol) is then added and the reaction mixture is stirred overnight at room temperature. The reaction is then heated at 150°C for 1 hour. The solvent is then removed and the residue is partitioned between water and methylene chloride. The organic layer is washed with water, dried over magnesium sulfate and evaporated to 11.5g of crude product. This crude product is extracted with 100ml hot hexane which affords 3.5g of white crystals. A final recrystallization from methanol affords 2.5g of white crystals of the intermediate ether ester (21% yield, m.p. 76-78°C).

The ether ester (2.2g, 5. 56 mmol) is hydrolyzed using the method of Example 1. Recrystallization from ethanol affords 1.1g of white crystals (65% yield, m.p. 149-151°C).

Analysis for: $C_{20}H_{18}O_3$
Calculated: C, 78.40; H, 5.92.
Found: C, 78.31; H, 6.02.

Example 3

α-Methyl-6-[(1-methyl-1H-benzimidazol-2-yl)-methoxy ]-2-naphthaleneacetic acid

The procedure of Example 2 is followed on a 10mmol scale, substituting 2-chlormethyl-N-methylbenzimidazole for benzyl chloride. Normal workup affords 2.5g of white crystals of ether ester (50% yield, m.p. 228-230°C). This material is analytically pure and needs no recrystallization.

The ether ester (2.5g, 4.9 mmol) is hydrolyzed according to the method of Example 1.

Recrystallization from ethanol affords 0.75g of white crystals (43% yield, m.p. 216-219°C).

Analysis for: $C_{22}H_{20}N_2O_3.3/4\ H_2O$
Calculated: C, 70.66; H, 5.79; N, 7.49.
Found: C, 70.89; H, 5.72; N, 7.28.

Example 4

α-Methyl-6-(2-pyridinylmethoxy)-2-naphthaleneacetic acid

To a solution of 6-hydroxy-α-methyl-2-naphthaleneacetic acid (10.8g, 50mmol) in methanol (100ml) is added sodium methoxide (100mmol). After 10 minutes the solvent is removed and replaced with hexamethylphosphoric triamide (250ml). 2-Chloromethyl-pyridine (100 mmol) is then added and the reaction is stirred for 5 days. The reaction is worked up by partitioning between water and methylene chloride, and the organic extract is evaporated to a crude oil which is chromatographed on silica gel (eluant: methylene chloride - ethyl acetate) to afford 6.0g of ether ester as an oil.

The ether ester is hydrolyzed using the method of Example 1. Recrystallization from ethanol affords 5.5g of white crystals (36% yield, m.p. 185-187°C).
Analysis for: $C_{19}H_{17}NO_3$
Calculated: C, 74.24; H,5.57; N, 4.56.
Found: C, 74.49; H,5.63; N, 4.49.

Example 5

α-Methyl-6-(2-benzothiazolylmethoxy)-2-naphthaleneacetic acid

The title compound is prepared according to the method of Example 1, using 2-(chloromethyl)-benzothiazole. White crystals are obtained having a melting point of 169-171°C.
Analysis for: $C_{21}H_{17}NO_3S.1/4H_2O$
Calculated: C, 68.55;H,4.79;N,3.80.
Found: C, 68.44;H,4.89;N,4.21.

Example 6

α-Methyl-6-(2-naphthalenylmethoxy)-2-naphthaleneacetic acid

The title compound is prepared according to the method of Example 1 using 2-(chloromethyl)-naphthalene. White crystals are obtained having a melting point of 216-218°C.
Analysis for: $C_{24}H_{20}O_3$
Calculated: C,80.88;H,5.66.
Found:C,80.68;H,5.89.

Example 7

S-(+)-α-Methyl-6-(2-quinolinylmethoxy)-2-naphthaleneace tic acid

To a solution of S-(+)-α-methyl-6-hydroxy-2-naphthalene acetic acid (prepared according to the procedure described in J. Med., 17, 377 (1974) (21.6g, 100 mmol) in methanol (250 ml) is added sodium methoxide (200 mmol). The solvent is removed in vacuo and replaced with dimethylformamide (300 ml). To this solution is added 2-(chloromethyl)quinoline (17.7 g, 100 mmol). After 90 minutes, the solvent is removed in vacuo at 50°C and the residue is partitioned between ethyl aetate and pH= 4 buffer. The insolubles and the ethyl acetate are heated to 60°C at which point a homogeneous solution is obtained. Cooling the solution to room temperature affords 8.9 g of white crystals (26%). A second recrystallization of 4.0 g of this material from methanol (200 ml) affords 1.78 g of white crystals, m.p. 192-194° C.
Analysis for: $C_{23}H_{19}NO_3$
Calculated: C,77.30;H,5.36;N,3.92.
Found: C,76.96;H,5.44;N,3.89
$(\alpha)_D$ =+51.7(Pyr c = 1.115)

Example 8

R-(-)-α-Methyl-6-(2-quinolinylmethoxy)-2-naphthaleneace tic acid

The title compound can be prepared according to the method of Example 7, using R-(-)-α-methyl-6-hydroxy-2-naphthalene acetic acid. White crystals are obtained having a melting point of 190-193°C.

Analysis for: $C_{23}H_{19}NO_3$
Calculated: C,77.30;H, 5.36; N, 3.92.
Found: C,76.87:H,5.54: N,3.77.
$(\alpha)_D$= -52.96(Pyr c = 1.08)

Example 9

α-Methyl-6-[(2-phenyl-4-thiazolyl)methoxy]-2-naphthalen eacetic acid

The title compound is prepared according to the method of Example 7 using 2-phenyl-4-(chloromethyl)-thiazole and racemic 6-hydroxy-α-methyl-2-naphthalene acetic acid. White crystals are obtained having a melting point of 163-164°C.
Analysis for: $C_{23}H_{19}NO_3S$
Calculated: C,70.93;H,4.92;N,3.60
Found: C,70.75;H, 4.77;N,3.32.

Example 10

α-Methyl-6-(dodecyloxy)-2-naphthaleneacetic acid

The title compound is prepared according to the method of Example 7 using 1-iododecane and racemic 6-hydroxy-α-methyl-2-naphthalene acetic acid. White crystals are obtained having a melting point of 96-97°C.
Analysis for: $C_{25}H_{36}O_3$
Calculated: C,78.08; H, 9.44.
Found: C, 78.10; H, 9.77.

Example 11

α-Methyl-6-(3-methyl-2-butenoxy)-2-naphthaleneacetic acid

The title compound is prepared according to the method of Example 7 using 1-bromo-3-methyl-2-butene and racemic 6-hydroxy-α-methyl-2-naphthalene acetic acid. White crystals are obtained having a melting point of 97-100°C.
Analysis for: $C_{18}H_{20}O_3$
Calculated: C, 76.03;H, 7.09
Found: C, 76.38; H, 7.10.

Example 12

α-Methyl-6-(3,3-dichloroallyloxy)-2-naphthaleneacetic acid

The title compound is prepared according to the method of Example 7 using 3,3-dichloroallyl chloride and racemic 6-hydroxy-α-methyl-2-naphthalene acetic acid. White crystals are obtained having a melting point of 107-109°C.
Analysis for: $C_{16}H_{14}O_3Cl_2$
Calculated: C, 59.10; H, 4.34.
Found: C, 59.43; H, 4.50.

Example 13

The compounds 5- and 12-hydroxyeicosatetraenoic acid (5-HETE and 12-HETE) and 5,12-dihydroxyeicosatetraenoic acid (5,12-diHETE) are early arachidonic acid oxidation products in the lipoxygenase cascade, which have been shown to mediate several aspects of inflammatory and allergic response. The assay of this Example measures the ability of the compounds of the invention to inhibit the synthesis of 5-HETE by rat glycogen elicited polymorphonuclear leukocytes.

The assay is carried out as follows:

Peritoneal PMN are obtained from female Wistar rats (150-250g) that received an i.p. injection of 6 glycogen (10ml). After 24 hours, rats are killed by $CO_2$ asphyxiation and peritoneal cells are harvested by peritoneal lavage using $Ca^{++}$ and $Mg^{++}$ free Hanks' balanced salt solution (HBSS). The peritoneal exudate is centrifuged at 400g for 10 minutes. After centrifugation, the lavaged fluid is removed and the cell pellet is resuspended in HBSS containing $Ca^{++}$ and $Mg^{++}$ and 10 mM L-cysteine at a concentration of $2 \times 10^7$ cells/ml. To 1 ml portions of cell suspension, test drugs or vehicle are added and incubated at 37°C for 10 minutes. Following this preincubation, the calcium ionophore (10 μM), A23187, is added together

with 0.5 μCi [$_{14}$C] arachidonic acid and further incubated for 10 minutes. The reaction is stopped by the addition of ice cold water (3 ml) and acidifying to pH 3.5. Lipoxygenase products are then extracted twice into diethyl ether. The pooled ether extracts are evaporated to dryness under nitrogen and the residue is redissolved in a small volume of methanol and spotted on aluminium backed pre-coated thin layer chromatographic plates. The samples are then cochromatographed with authentic reference 5-HETE in the solvent system - hexane : ether : acetic acid (50:50:3). After chromatography, the areas associated with 5-HETE standard are identified by autoradiography, cut out and quantitated by liquid scintillation

The compounds of the invention and the nonsterioidal anti-inflammatory drug naproxen, when tested in this assay at the level of 10 μM, gave the following results in inhibiting the synthesis of the arachidonic acid lipoxygenase oxidation product 5-HETE.

Table 1

| Compound of Example No. | % Inhibition of 5-LO (as 5-HETE) |
|---|---|
| naproxen | –94* |
| 1 | 100 |
| 2 | 84 |
| 3 | 24 |
| 4 | 82 |
| 5 | 90 |
| 6 | 100 |
| 7 | 100 |
| 8 | 33** |
| 9 | 86 |
| 11 | 81 |

* The negative value denotes a potentiation of 5-HETE synthesis.
** Tested at a level of 0.1 μM.

These results show that the compounds of the invention exhibit very significant activity in inhibiting the enzyme, lipoxygenase.

Example 14

The procedure of Example 13 is also employed for the determination of the ability of the compounds of the invention to inhibit the synthesis of the arachidonic acid cyclooxygenase oxidation product $TxB_2$.

In this assay, the procedure of Example 13 is carried out as described. However, in order to determine cyclooxygenase activity, the samples are cochromatographed with authentic reference $TxB_2$ and $PGE_2$ in the solvent system ethyl acetate : formic acid (80 : 1) and the upper phase of ethyl acetate : isooctane:acetic acid : water (110 : 50 : 20 : 100). After chromatography, the areas associated with the $TxB_2$ and $PGE_2$ standards are indentified by autoradiography, cut out and quantitated by liquid scintillation techniques

The results are calculated as in Example 13.

When tested in this assay the compounds of the invention and the non-steroidal anti-inflammatory drug naproxen, a well-established inhibitor of cyclooxygenase, at a level of 10 μM gave the following results in inhibiting the synthesis of the arachidonic acid cyclooxygenase oxidation product $TxB_2$ and $PGE_2$

Table 2

| Compound of Example No. | % Inhibitor of CO (as $TxB_2$) | % Inhibitor of CO (as $PGE_2$) |
|---|---|---|
| naproxen | | 85 |
| 1 | 28 | –37* |
| 2 | 6 | –26* |
| 3 | 15 | –29* |
| 4 | 12 | 18 |
| 5 | | –35* |
| 6 | | –15* |
| 7 | | –17* |
| 8 | | –7*, ** |
| 9 | | –11* |
| 11 | | –15* |

* The negative values denote a potentiation of $PGE_2$ synthesis.
** Tested at level of 0.1 µM.

These results show that the compounds of the invention, in contradistinction to naproxen, are virtually devoid of cyclooxygenase inhibitory activity, having activity substantially only on the lipoxygenase pathway of arachidonic acid oxidation.

Example 15

The assay of this Example measures the in vivo ability of the compounds of the invention to inhibit the bronchospasm induced in guinea pigs by the exogenously administered leukotrienes $C_4$ and /or $D_4$.

This assay is carried out as follows:

Male Hartley strain guinea pigs (350-600g) are anesthetized with pentobarbital sodium (50 mg/kg, i.p.). The jugular vein is cannulated for injection of drugs and the carotid artery for monitoring blood pressure. The trachea is cannulated for artificial ventilation by a miniature Starling pump and for indirect measurement of respiratory volume changes. The animals are then pretreated with succinylcholine (2 mg/kg i.v.) and indomethacin (10 mg/kg i.v. in trizma 8.3 buffer, 9 minutes prior to leukotriene challenge). Submaximal bronchoconstrictor responses are established in control animals by varying the dose-levels of leukotriene. Intravenous dose-levels for $LTC_4$ range from 0.4 to 0.6 µg/kg and for $LTD_4$ the range is from 0.3 to 0.5 µg/kg. The aerosol bronchoprovocation dose for $LTC_4$ is generated from 1.6µM solution and for $LTD_4$ from a 2.0 µM solution.

Test drugs (dissolved in a solvent such as propylene glycol, polyethylene glycol 400 or saline) are administered either intraduodenally, by aerosol or intragastrically at 2 or 10 minutes before induction of bronchospasm by administration of either $LTC_4$ or $LTD_4$ at the predetermined dose-levels. Aerosols of soluble drugs or leukotrienes are produced in-line for 10 seconds only by actuation of an ultrasonic nebulizer (Monaghan). Aerosolized drug dosage is expressed in terms of solution concentration and by a fixed aerosol exposure time (approximately 10 seconds). Control animals receive solvent (2 ml/kg i.d. or appropriate aerosol) in place of drug.

Respiratory volume changes are determined by a calibrated piston whose travel is recorded, via a linear transducer, on a Beckman Dynograph recorder. Maximal bronchoconstrictor volume is determined by clamping off the trachea at the end of the experiment. Overflow volumes at 1, 3 and 5 minutes are obtained from the recorded charts.

Area under the volume overflow curve (AUC) is estimated, using the overflow values at 1,3 and 5 minutes, and expressed as a percentage of the maximal overflow AUC (equation 1):

$$\%\ \max AUC = \frac{3\ (1\ min) + 4\ (3\ min) + 2\ (5\ min)}{10\ (max)} \times 100 \qquad 1)$$

Drug effects are reported as percent inhibition of % max AUC values obtained from appropriate control animals (equation 2):

(2

$$\% \text{ inhibition} = \frac{\% \text{ max AUC control} - \% \text{ max AUC treated}}{\% \text{ max AUC control}} \times 100$$

Student's t-test for unpaired data is used to determine statistical significance ($p < 0.05$). $IC_{50}$ values can also be determined by inverse prediction from linear regression lines through points between 10 and 90% inhibition.

The results for compounds of the invention are as follows:

Table 3

Compound administered at 10 minutes before induction of bronchospasm using $LTD_4$

| Compound of Example Number | Dose * mg/kg | % Inhibition |
|---|---|---|
| 1 | 25 | 75 |
| 2 | 25 | –76 |
| 3 | 25 | 80 |
| 4 | 25 | 52 |

* = intraduodenally administered

The results show that compounds of the invention have significant <u>in vivo</u> activity against LTD4 induced bronchoconstriction.

Example 16

The compounds of the invention are further tested in the rat carrageenan paw edem assay to determine their ability to inhibit the acute inflammatory response.

This assay is carried out as follows:

140–180 gm male Sprague-Dawley rats, in groups of 6 animals are injected subcutaneously in the right paw with 0.1ml of 1% carrageenan at zero time. Mercury plethysmographic readings (ml) of the paw are made at zero time and 3 hours later. Test compounds are suspended or dissolved in 0.5% methylcellulose and given perorally 1 hour prior to carrageenan administration.

The increase in paw volume (edema in ml.) produced by the carrageenan is measured. Paw edema is calculated (3 hour volume minus zero time volume), and percent inhibition of edema is determined. Unpaired Student's t-test is used to determine statistical significance.

The activity of standard drugs in this assay is as follows:

| Drug | Oral $ED_{50}$ (95% C.L.) mg/kg |
|---|---|
| Indomethacin | 3.7 (0.6, 23.8) |
| Aspirin | 145.4 (33.1, 645.6) |
| Phenylbutazone | 26.2 (2.3, 291.0) |

When tested in this assay, the compounds of the invention gave the following results:

Table 4

| Compound of Inhibition at Example No. | % 50 mg/kg (per oral) |
|---|---|
| 1 | 42 |
| 2 | 24 |
| 3 | 32 |
| 4 | 30 |

The results show that the compounds tested have activity in the rat carrageenan paw edema assay, evidencing an effect on the acute inflammatory response.

Example 17

The compounds of the invention are tested in the rat acute gastroirritation assay to examine their potential for causing gastric irritation when administered at doses exceeding the effective dose. The nonsteroidal anti-inflammatory drug naproxen is tested as a standard of a compound known to possess gastroirritant side effects.

This assay is carried out as follows:

Male Sprague Dawley rats (190–220 g) are fasted for 18 hours prior to drug administration. Rats are divided into groups of 8 and coded (i.e., observer of gastric lesions is not aware of drug treatment). Drugs were dissolved or suspended in 0.5% Tween 80 and administered by gastric intubation in a volume of 1 ml/100 g body weight, control rats receiving only Tween 80. Four hours after drug administration, rats are evaluated by recording the incidence and severity of gastroirritation using the following scoring system: 0) No irritation or lesions; 1) irritation (redness); 2) $\leq$ 5 lesions (ulcers) and 3) > 5 lesions. Dunnett's test ( $\alpha$= 0.05) was used to calculate the mean ± SE of each test group and the statistical significance.

The results of the assay are presented in Table 5.

Table 5

| Compound of Example No. | Dose mg/kg | % of rats with GI lesions |
|---|---|---|
| naproxen | 25 | 100 |
| 1 | 300 | 0 |

The results show the compounds of the invention to have little potential for acute gastroirritation when compared to naproxen.

Example 18

The assay of Example 17 is also used to measure the cytoprotective activity of the compounds against acute gastroirritation induced by a nonsteroidal anti-inflammatory drug (NSAID).

The assay is carried out as presented in Example 17 with the following modifications: one hour before administration of the NSAID, the rats perorally receive either vehicle (1 ml/100 g body weight of 0.5% Tween 80) or drug (100 mg/kg); at 0 hour the rats perorally receive 25 mg/kg of the NSAID naproxen or vehicle (1 ml/100 g body weight of 0.5% Tween 80); at 4 hours post-dosing the rats are sacrificed and gastroirritation assessed as described in Example 17.

The results are presented in Table 6.

Table 6

| Compound of Example No. | Dose mg/kg | % of Rats With Lesions | Comments |
|---|---|---|---|
| vehicle | | 12.5 | – |
| naproxen | 25 | 75.0 | – |
| 7 | 100 | 87.5 | no protection |

**Claims for the contracting states: AT, BE, CH, DE, FR, IT, LI, LU, NL, SE**

1. A compound having the formula:

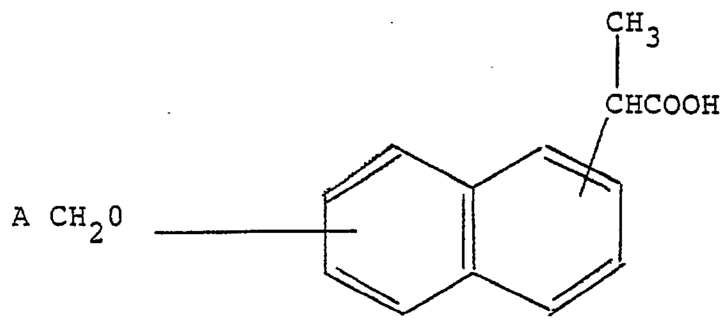

wherein A is alkyl of 3–19 carbon atoms, diloweralkyl vinyl, dihalovinyl, diphenylvinyl, lower alkynyl,

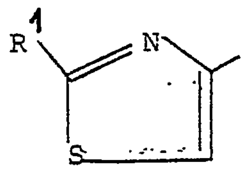 or 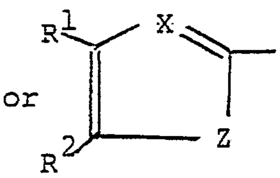 ;

X is N or CR;

Z is $-\underset{R}{C}=\underset{R}{C}-$, $-\underset{R}{C}=N-$, $-N=\underset{R}{C}-$, $-\underset{R}{N}-$, S or O;

R is hydrogen or lower alkyl
$R_1$ is hydrogen, lower alkyl or phenyl;
$R_2$ is hydrogen or lower alkyl; or
$R_1$ and $R_2$ taken together with the carbon atoms to which they are attached form a benzene ring; or a pharmaceutically acceptable salt thereof, with the proviso that A is not 3 to 5 carbon atoms when $ACH_2O$ is in the 6-position.

2. A compound of formula I as claimed in claim 1, wherein A is

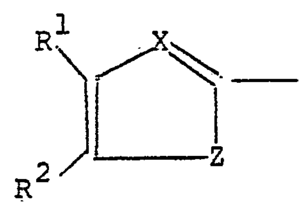

X is CR, Z is

$$-N=\underset{R}{C}-$$

and $R^1$ and $R^2$ taken together with the carbon atoms to which they are attached form a benzene ring.

3. A compound of formula I as claimed in claim 1, wherein A is

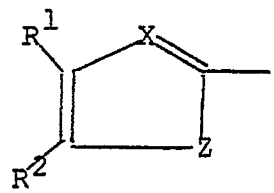

X is CR, Z is

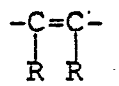

and R¹ and R² are hydrogen.

4. A compound of formula I, as claimed in claim1, wherein A is

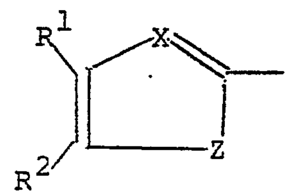

X is N, Z is

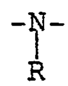

and R¹ and R² taken together with the carbon atoms to which they are attached form a benzene ring.

5. A compound of formula I, as claimed in claim 1, wherein A is

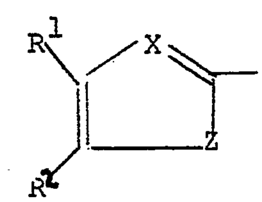

X is N, Z is

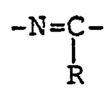

and R¹ and R² are hydrogen.

6. α-Methyl-6-(2-quinolinylmethoxy)-2-naphthaleneacetic acid or a pharmaceutically acceptable salt thereof.

7. α-Methyl-6-(phenylmethoxy)-2-naphthaleneacetic acid or a pharmaceutically acceptable salt thereof.

8. α-Methyl-6-[(1-methyl-1H-benzimidazol-2-yl)methoxy]2-naphthaleneacetic acid cr a pharmaceutically acceptable salt thereof.

9. α-Methyl-6-(2-pyridinylmethoxy)-2-naphthaleneacetic acid or a pharmaceutically acceptable salt thereof.

10. α-Methyl-6-(2-benzothiazolylmethoxy)-2-naphthaleneacetic acid or a pharmaceutically acceptable salt thereof.

11. α-Methyl-6-(2-naphthalenylmethoxy)-2-naphthaleneacetic acid or a pharmaceutically acceptable salt thereof.

12. S-(+)-α-methyl-6-(2-quinolinylmethoxy)-2-naphthaleneacetic acid,
R-(-)-α-methyl-6-(2-quinolinylmethoxy)-2-naphthaleneacetic acid, or a pharmaceutically acceptable salt thereof.

13. α-Methyl-6-[(2-phenyl-4-thiazolyl)-methoxy]-2-naphthaleneacetic acid or a pharmaceutically acceptable salt thereof.

14. α-Methyl-6-(dodecyloxy)-2-naphthaleneacetic acid, α-methyl-6-(3-methyl-2-butenoxy)-2-naph-

thaleneacetic acid, or α-methyl-6-(3,3-dichloroallyloxy)-2-naphthaleneacetic acid; or a pharmaceutically acceptable salt thereof.

15. An alkali-metal salt of a compound as claimed in any one of the preceding claims.

16. An amine salt of a compound as claimed in any one of claims 1–14.

17. α-Methyl-6-(2-quinolinylmethoxy)-2-naphthaleneacetic acid sodium salt.

18. A pharmaceutical composition comprising a compound as claimed in any one of claims 1 to 17 and a pharmaceutically acceptable carrier.

19. A compound as claimed in any one of claims 1 to 17 for use as an anti-inflammatory, anti-allergy or cytoprotective agent.

20. A process for preparing a compound of formula I as claimed in claim 1, or a pharmaceutically acceptable salt thereof characterised in that an ether ester of formula IVa

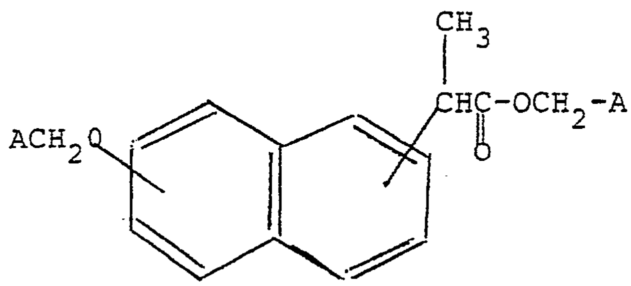

wherein A is as defined in claim 1 is hydrolyzed and if desired the compound of formula I is isolated as a pharmaceutically acceptable salt.

21. A process for preparing a compound of formula I as defined in claim 1 or a pharmaceutically acceptable salt thereof, characterised in that a compound of formula II

$ACH_2$–Hal II

wherein A is as defined in claim 1 and Hal is a halogen atom selected from chlorine, bromine and iodine is reacted with a dimetal derivative of a compound of formula III

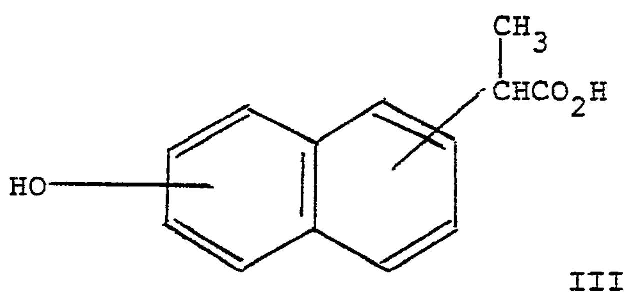

III

and if desired the product of formula I is isolated as a pharmaceutically acceptable salt.

22. A compound of formula IVa or a pharmaceutically acceptable salt thereof

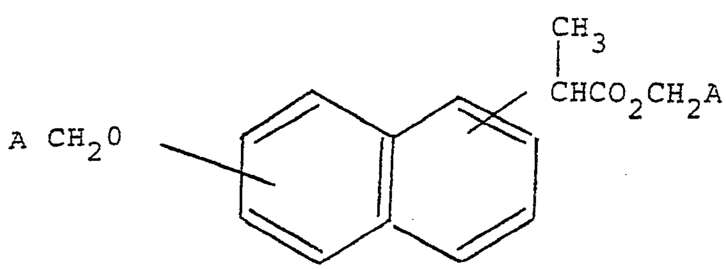

wherein A is as defined in claim 1.

23. A compound of formula IV, or a pharmaceutically acceptable salt thereof

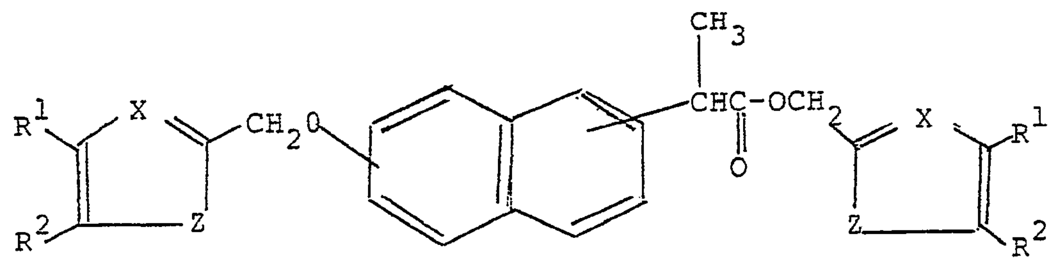

wherein $R^1$, $R^2$, X and Z are as defined in claim 1.

24. A process for preparing a compound of formula I as claimed in claim 1, or a pharmaceutically acceptable salt thereof characterised in that an ester of formula IVb

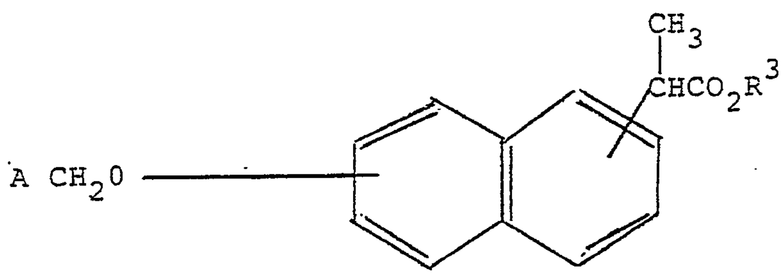

wherein A is defined in claim 1 and $R^3$ is an alkyl group of 1–6 carbon atoms is hydrolysed and if desired, the product of formula I is isolated as a pharmaceutically acceptable salt.

**Claims for the contracting states: ES, GR**

1. A process for preparing a compound having the formula:

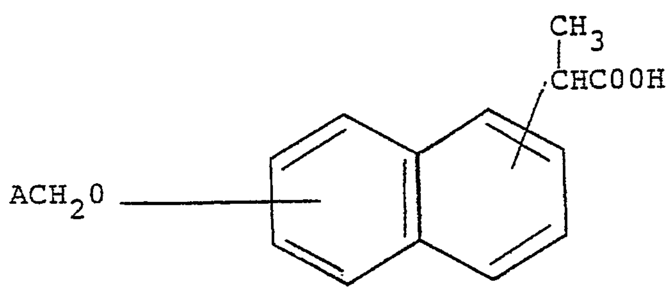

wherein A is alkyl of 3–19 carbon atoms, diloweralkyl vinyl, dihalovinyl, diphenylvinyl, lower alkynyl

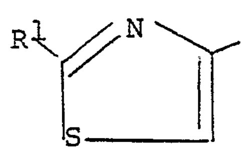

or

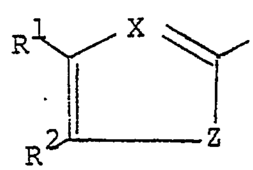

X is N or CR;

Z is 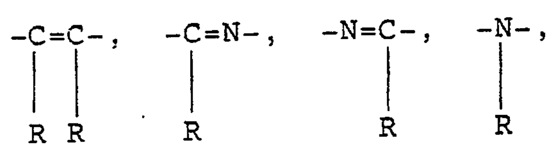 S or O;

R is hydrogen or lower alkyl;
$R^1$ is hydrogen lower alkyl or phenyl;
$R^2$ is hydrogen or lower alkyl; or
$R^1$ and $R^2$ taken together with the carbon atoms to which they are attached form a benzene ring;

or a pharmaceutically acceptable salt thereof, characterised in that an ether ester of formula(IVa)

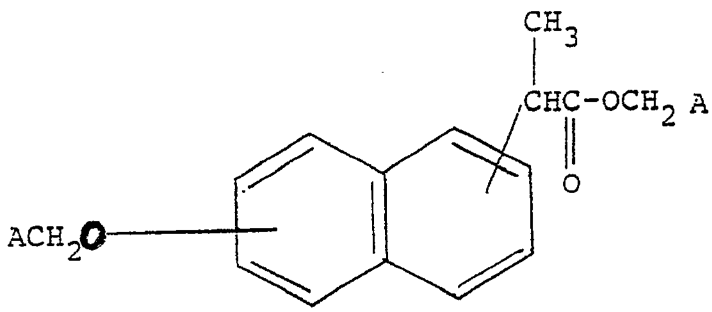

wherein A is as defined above, is hydrolyzed to obtain a compound of formula I, which if desired is isolated as a pharmaceutically acceptable salt with the proviso that A is not 3–5 carbon atoms when $ACH_2O$ is in the 6-position.

2. A process as claimed in claim 1, characterised in that the starting material of formula IV is prepared by reacting a compound of formula II

$ACH_2Hal$

II

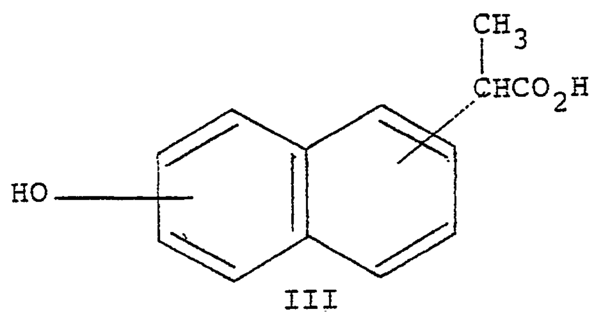

III

wherein A is as defined above and Hal is a halogen atom selected from chlorine, bromine and iodine, with a metal derivative of a compound of formula III.

3. A process for preparing a compound of formula I as defined in claim 1, characterised in that a compound of formula II

$ACH_2Hal$

II

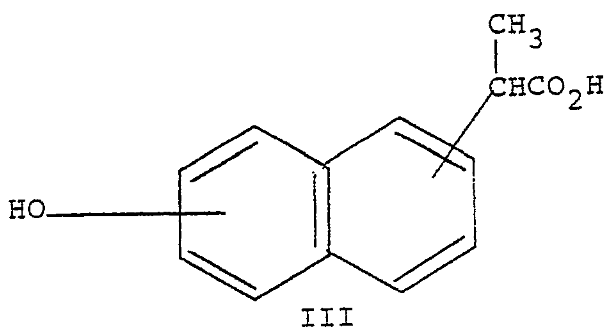

III

wherein A is as defined above and Hal is a halogen atom selected from chlorine, bromine and iodine, is reacted with a dimetal derivative of a compound of formula III and if desired the product is isolated as a pharmaceutically acceptable salt.

4. A process as claimed in claim 2, or claim 3 characterised in that the metal derivative of the compound of formula III is prepared by treating the compound of formula III with an alkali metal alkoxide.

5. A process for preparing a compound of formula I as defined in claim 1, or a pharmaceutically acceptable salt thereof, characterised in that an ester of formula IVb

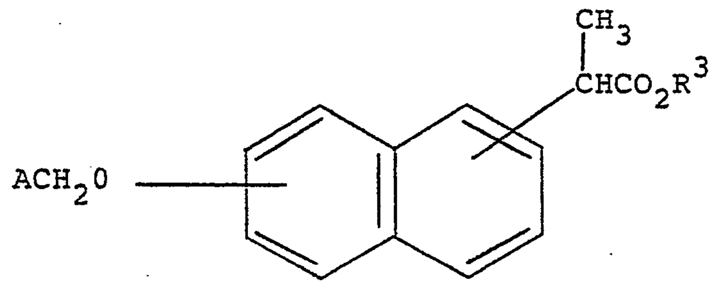

wherein A is as defined in claim 1, and $R^3$ is an alkyl group of 1 to 6 carbon atoms, is hydrolysed and if desired the product of formula I is isolated as a pharmaceutically acceptable salt.

6. A process as claimed any one of claims 1–5, characterised in that a starting material is used in which A is

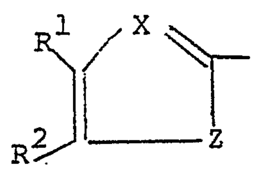

X is CR, Z is

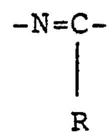

and $R^1$ and $R^2$ taken together with the carbon atoms to which they are attached form a benzene ring.

7. A process as claimed in any one of claims 1–5, characterised in that a starting material is used in which A is

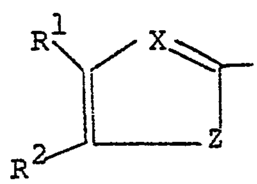

X is CR, Z is

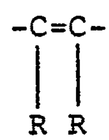

and $R^1$ and $R^2$ are hydrogen.

8. A process as claimed in any one of claims 1–5, characterised in that a starting material is used in which A is

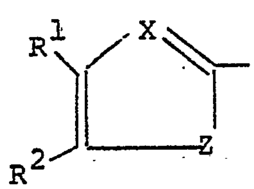

X is N, Z is

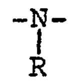

and $R^1$ and $R^2$ taken together with the carbon atoms to which they are attached form a benzene ring.

9. A process as claimed in any one of claims 1–5, characterised in that a starting material is used in which A is

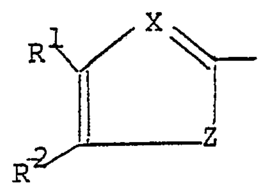

X is N, Z is

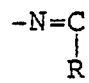

and $R^1$ and $R^2$ are hydrogen.

10. A process as claimed in anyone of claims 1–5, characterised in that a starting material is used in which A is alkyl of 3–19 carbon atoms.

11. A process as claimed in any one of claims 1–5, characterised in that a starting material is used in which A is 2-benzothiazolyl.

12. A process as claimed in any one of claims 1–5, characterised in that a starting material is used in which A is 2-naphthalenyl or 2-quinolinyl.

13. A process as claimed in any one of claims 1–5, characterised in that a starting material is used in which A is 2-phenyl-4-thiazolyl.

14. A process as claimed in any one of claims 1–5, characterised in that a starting material is used in which A is 2, 2-dichlorovinyl or 2, 2-dimethylvinyl.

15. A process for preparing a pharmaceutically acceptable salt of a compound of formula I as defined in claim 1, which process comprises treating a compound of formula I containing at least one nitrogen atom with a pharmaceutically acceptable acid to form an acid addition salt or treating a compound of formula I as defined in claim 1, with a base of a pharmaceutically acceptable cation to form a salt of the carboxylic acid function of the compound of formula I.

16. A process as claimed in claim 15, characterised in that a compound of formula I as defined in claim 1, is reacted with an alkali-metal base to form an alkali-metal salt of the carboxylic acid function.

17. A process as claimed in claim 15, characterised in that a compound of formula I as defined in claim 1 is reacted with an amine to form an amine salt of the carboxylic acid function.

18. A process for preparing a pharmaceutical composition characterised in that a compound for formula I as defined in claim 1, or a pharmaceutically acceptable salt thereof, is mixed with a pharmaceutical carrier and the resulting composition is brought into a form suitable for therapeutic administration.

19. A process as claimed in claim 18, characterised in that the compound of formula I is prepared by a process as claimed in any one of claims 1 to 14.

20. The use of a compound of formula I is defined in claim 1, or a pharmaceutically acceptable salt thereof, for the manufacture of a medicament for use as an anti-inflammatory, anti-allergy or cytoprotective agent.

21. A process for preparing an ether ester of formula IV as defined in claim 1 characterised in that a compound of formula II

$ACH_2Hal$

II

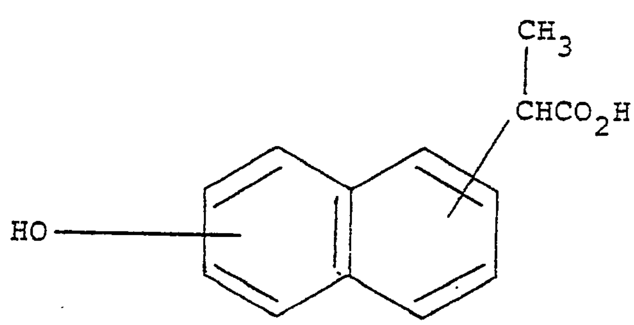

III

wherein A is as defined in claim I and Hal is a halogen atom selected from chlorine, bromine and iodine, is reacted with a dimetal derivative of a compound of formula III.

22. A process as claimed in claim 21 characterised in that the metal derivative of the compound of formula III is a dialkali metal derivative.

**Patentansprüche für die benannten Vertragsstaaten: AT, BE, CH, DE, FR, IT, LI, LU, NL, SE**

1. Verbindung der Formel

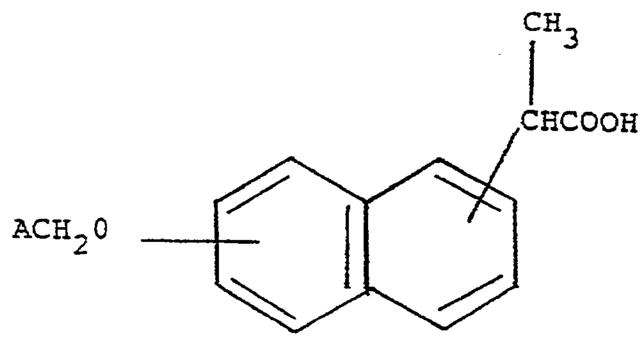

,

worin A Alkyl mit 3 bis 19 Kohlenstoffatomen, Di-nied.alkylvinyl, Dihalogenvinyl, Diphenylvinyl, nied.Alkinyl,

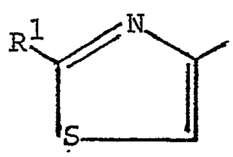

oder

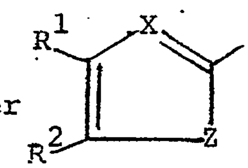

bedeutet; X N oder CR ist; Z

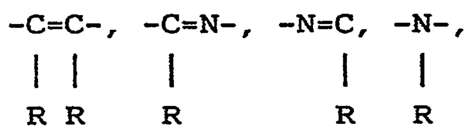

S oder O darstellt; R Wasserstoff oder nied.Alkyl bedeutet; $R^1$ Wasserstoff, nied.Alkyl oder Phenyl ist; $R^2$ die Bedeutung Wasserstoff oder nied.Alkyl hat; oder $R^1$ und $R^2$ zusammen mit den Kohlenstoffatomen, an die sie gebunden sind, einen Benzolring bilden; oder ein pharmazeutisch annehmbares Salz hievon, mit der Maßgabe, daß A nicht 3 bis 5 Kohlenstoffatome aufweist, wenn $ACH_2O$ in Stellung 6 ist.

2. Verbindung der Formel (I), wie in Anspruch 1 beansprucht, worin A

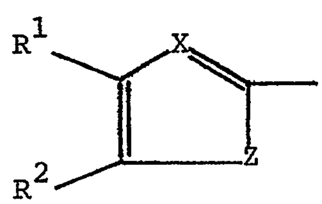

ist, X die Bedeutung CR hat, Z

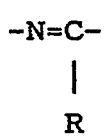

ist und $R^1$ und $R^2$ zusammen mit den Kohlenstoffatomen, an die sie gebunden sind, einen Benzoring bilden.

3. Verbindung der Formel (I), wie in Anspruch 1 beansprucht, worin A

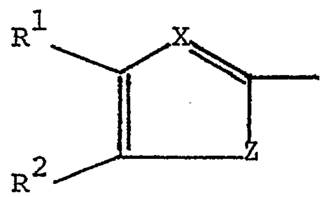

ist, X die Bedeutung CR hat, Z

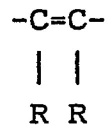

ist und $R^1$ und $R^2$ Wasserstoff darstellen.

4. Verbindung der Formel (I), wie in Anspruch 1 beansprucht, worin A

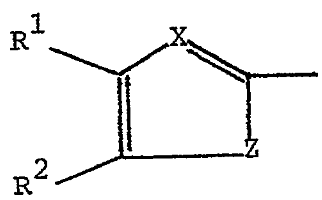

ist, X die Bedeutung N hat, Z

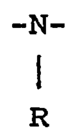

ist und $R^1$ und $R^2$ zusammen mit den Kohlenstoffatomen, an die sie gebunden sind, einen Benzolring bilden.

5. Verbindung der Formel (I), wie in Anspruch 1 beansprucht, worin A

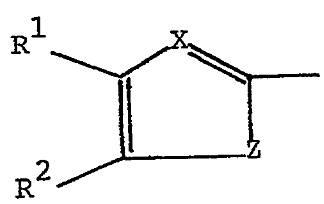

ist, X die Bedeutung N hat, Z

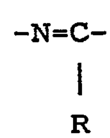

ist und $R^1$ und $R^2$ Wasserstoff darstellen.

6. α-Methyl-6-(2-chinolinylmethoxy)-2-naphthalinessigsäure oder ein pharmazeutisch annehmbares Salz hievon.

7. α-Methyl-6-(phenylmethoxy)-2-naphthalinessigsäure oder ein pharmazeutisch annehmbares Salz hievon.

8. α-Methyl-6-[(1-methyl-lH-benzimidazol-2-yl)-methoxy]-2-naphthalinessigsäure oder ein pharmazeutisch annehmbares Salz hievon.

9. α-Methyl-6-(2-pyridinylmethoxy)-2-naphthalinessigsäure oder ein pharmazeutisch annehmbares Salz hievon.

10. α-Methyl-6-(2-benzothiazolylmethoxy)-2-naphthalinessigsäure oder ein pharmazeutisch annehmbares Salz hievon.

11. α-Methyl-6-(2-naphthalenylmethoxy)-2-naphthalinessigsäure oder ein pharmazeutisch annehmbares Salz hievon.

12. S-(+)-α-Methyl-6-(2-chinolinylmethoxy)-2-naphthalinessigsäure, R-(–)-α-Methyl-6-(2-chinolinylmethoxy)-2-naphthalinessigsäure oder ein pharmazeutisch annehmbares Salz hievon.

13. α-Methyl-6-[(2-phenyl-4-thiazolyl)-methoxy]-2-naphthalinessigsäure oder ein pharmazeutisch annehmbares Salz hievon.

14. α-Methyl-6-(dodecyloxy)-2-naphthalinessigsäure, α-Methyl-6-(3-methyl-2-butenoxy)-2-naphthalinessigsäure oder α-Methyl-6-(3,3-dichlorallyloxy)-2-naphthalinessigsäure oder ein pharmazeutisch annehmbares Salz hievon.

15. Alkalimetallsalz einer Verbindung, wie in einem der vorhergehenden Ansprüche beansprucht.

16. Aminsalz einer Verbindung, wie in einem der Ansprüche 1 bis 14 beansprucht.

17. α-Methyl-6-(2-chinolinylmethoxy)-2-naphthalinessigsäurenatriumsalz.

18. Pharmazeutische Zusammensetzung umfassend eine Verbindung, wie in einem der Ansprüche 1 bis 17 beansprucht, und einen pharmazeutisch annehmbaren Träger.

19. Verbindung, wie in einem der Ansprüche 1 bis 17 beansprucht, zur Verwendung als entzündungshemmendes, antiallergisches oder zellenschützendes Mittel.

20. Verfahren zum Herstellen einer Verbindung der Formel (I), wie in Anspruch 1 beansprucht, oder eines pharmazeutisch annehmbaren Salzes hievon, dadurch gekennzeichnet, daß ein Ätherester der Formel (IVa)

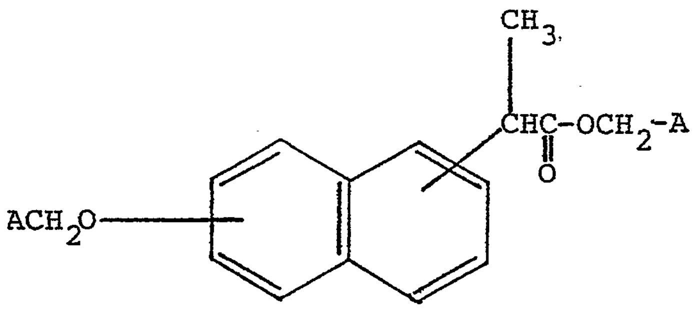

,

worin A wie in Anspruch 1 definiert ist, hydrolysiert und, wenn gewünscht, die Verbindung der Formel (I) als ein pharmazeutisch annehmbares Salz isoliert wird.

21. Verfahren zum Herstellen einer Verbindung der Formel (I), wie in Anspruch 1 definiert, oder eines pharmazeutisch annehmbaren Salzes hievon, dadurch gekennzeichnet, daß eine Verbindung der Formel (II)

$ACH_2$-Hal (II),

worin A wie in Anspruch 1 definiert ist und Hal ein Halogenatom ausgewählt aus Chlor, Brom und Jod ist, mit einem Dimetallderivat einer Verbindung der Formel (III)

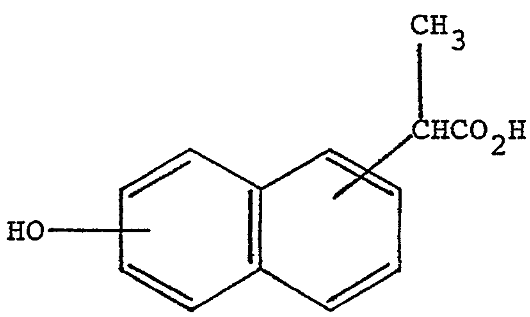

(III)

umgesetzt und, wenn gewünscht, das Produkt der Formel (I) als pharmazeutisch annehmbares Salz isoliert wird.

22. Verbindung der Formel (IVa) oder ein pharmazeutisch annehmbares Salz hievon

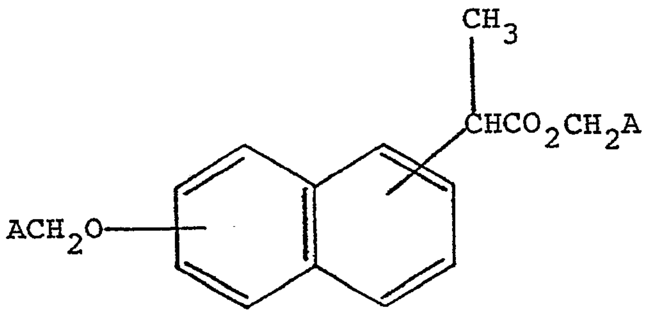

,

worin A wie in Anspruch 1 definiert ist.

23. Verbindung der Formel (IV) oder ein pharmazeutisch annehmbares Salz hievon

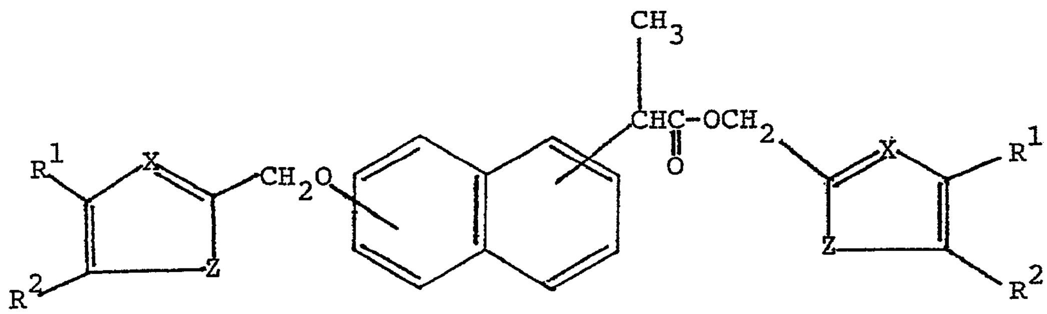

,

worin $R^1$, $R^2$, X und Z wie in Anspruch 1 definiert sind.

24. Verfahren zum Herstellen einer Verbindung der Formel (I), wie in Anspruch 1 beansprucht, oder eines pharmazeutisch annehmbaren Salzes hievon, dadurch gekennzeichnet, daß ein Ester der Formel (IVb)

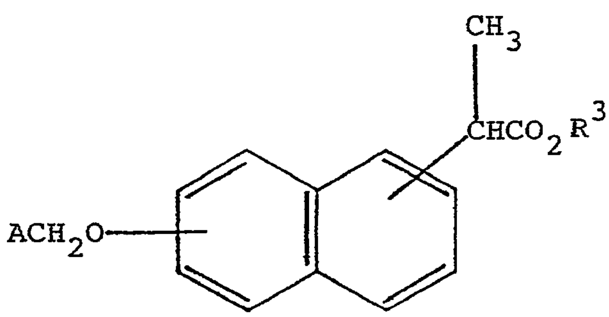

,

worin A wie in Anspruch 1 definiert ist und $R^3$ eine Alkylgruppe mit 1 bis 6 Kohlenstoffatomen ist, hydrolysiert und, wenn gewünscht, das Produkt der Formel (I) als pharmazeutisch annehmbares Salz isoliert wird.

**Patentansprüche für die benannten Vertragsstaaten: ES, GR**

1. Verfahren zum Herstellen einer Verbindung der Formel

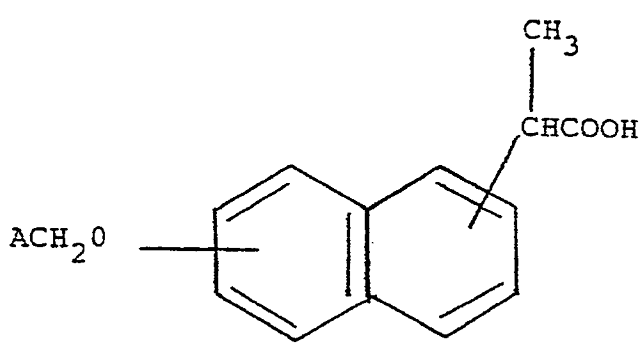

,

worin A Alkyl mit 3 bis 19 Kohlenstoffatomen, Di-nied.alkylvinyl, Dihalogenvinyl, Diphenylvinyl, nied.Alkinyl,

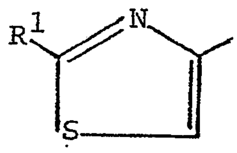

oder

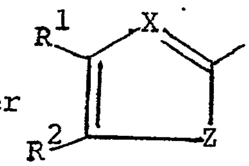

bedeutet; X N oder CR ist; Z

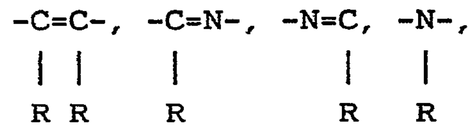

S oder O darstellt; R Wasserstoff oder nied.Alkyl bedeutet; $R^1$ Wasserstoff, nied.Alkyl oder Phenyl ist; $R^2$ die Bedeutung Wasserstoff oder nied.Alkyl hat; oder $R^1$ und $R^2$ zusammen mit den Kohlenstoffatomen, an die sie gebunden sind, einen Benzolring bilden; oder eines pharmazeutisch annehmbaren Salzes hievon, dadurch gekennzeichnet, daß ein Ätherester der Formel (IV)

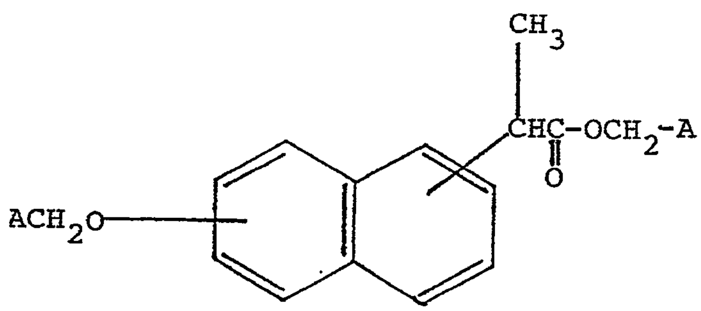

,

worin A wie oben definiert ist, hydrolysiert wird, um eine Verbindung der Formel (I) zu erhalten, die, wenn gewünscht, als pharmazeutisch annehmbares Salz isoliert wird, mit der Maßgabe, daß A nicht 3 bis 5 Kohlenstoffatome aufweist, wenn $ACH_2O$ in Stellung 6 ist.

2. Verfahren, wie in Anspruch 1 beansprucht, dadurch gekennzeichnet, daß das Ausgangsmaterial der Formel (IV) hergestellt wird durch Umsetzen einer Verbindung der Formel (II)

$ACH_2Hal$

(II)

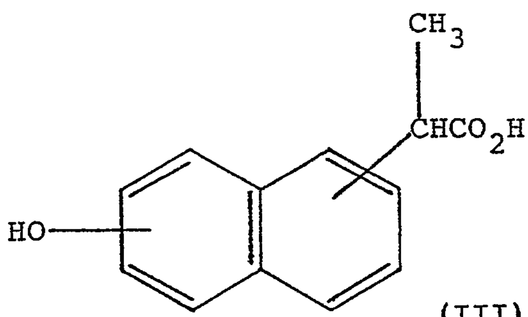

(III)

,

worin A wie oben definiert ist und Hal ein Halogenatom ausgewählt aus Chlor, Brom und Jod bedeutet, mit einem Metallderivat einer Verbindung der Formel (III).

3. Verfahren zum Herstellen einer Verbindung der Formel (I), wie in Anspruch 1 definiert, dadurch gekennzeichnet, daß eine Verbindung der Formel (II)

$ACH_2Hal$ 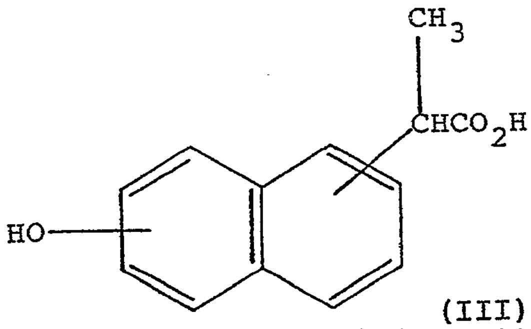

(II) (III)

worin A wie oben definiert ist und Hal ein Halogenatom ausgewählt aus Chlor, Brom und Jod bedeutet, mit einem Dimetallderivat einer Verbindung der Formel (III) umgesetzt und, wenn gewünscht, das Produkt als pharmazeutisch annehmbares Salz isoliert wird.

4. Verfahren, wie in Anspruch 2 oder Anspruch 3 beansprucht, dadurch gekennzeichnet, daß das Metallderivat der Verbindung der Formel (III) durch Behandeln der Verbindung der Formel (III) mit einem Alkalimetallalkoxid hergestellt wird.

5. Verfahren zum Herstellen einer Verbindung der Formel (I), wie in Anspruch 1 definiert, oder eines pharmazeutisch annehmbaren Salzes hievon, dadurch gekennzeichnet, daß ein Ester der Formel (IVb)

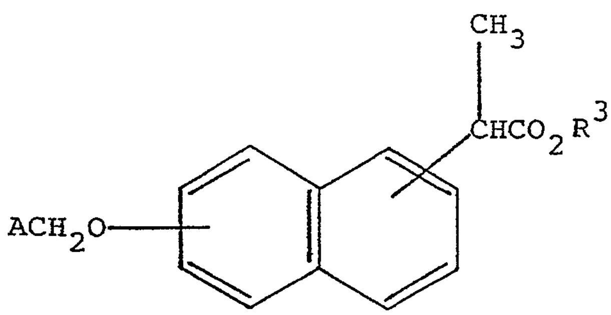

worin A wie in Anspruch 1 definiert ist und $R^3$ eine Alkylgruppe mit 1 bis 6 Kohlenstoffatomen bedeutet, hydrolysiert und, wenn gewünscht, das Produkt der Formel (I) als pharmazeutisch annehmbares Salz isoliert wird.

6. Verfahren, wie in einem der Ansprüche 1 bis 5 beansprucht, dadurch gekennzeichnet, daß ein Ausgangsmaterial verwendet wird, in dem A

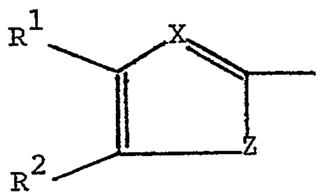

ist, X die Bedeutung CR hat, Z

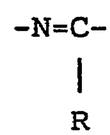

ist und $R^1$ und $R^2$ zusammen mit den Kohlenstoffatomen, an die sie gebunden sind, einen Benzolring bilden.

7. Verfahren, wie in einem der Ansprüche 1 bis 5, dadurch gekennzeichnet, daß ein Ausgangsmaterial verwendet wird, worin A

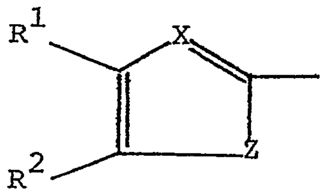

ist, X die Bedeutung CR hat, Z

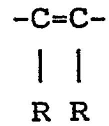

ist und $R^1$ und $R^2$ Wasserstoff darstellen.

8. Verfahren, wie in einem der Ansprüche 1 bis 5 beansprucht, dadurch gekennzeichnet, daß ein Ausgangsmaterial verwendet wird, worin A

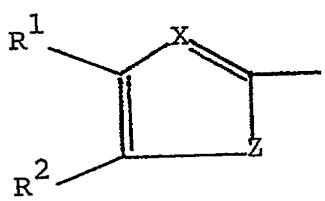

ist, X die Bedeutung N hat, Z

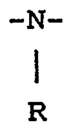

ist und $R^1$ und $R^2$ zusammen mit den Kohlenstoffatomen, an die sie gebunden sind, einen Benzolring bilden.

9. Verfahren, wie in einem der Ansprüche 1 bis 5 beansprucht, dadurch gekennzeichnet, daß ein Ausgangsmaterial verwendet wird, worin A

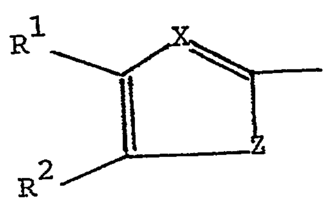

ist, X die Bedeutung N hat, Z

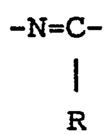

ist und $R^1$ und $R^2$ Wasserstoff darstellen.

10. Verfahren, wie in einem der Ansprüche 1 bis 5 beansprucht, dadurch gekennzeichnet, daß ein Ausgangsmaterial verwendet wird, worin A Alkyl mit 3 bis 19 Kohlenstoffatomen ist.

11. Verfahren, wie in einem der Ansprüche 1 bis 5 beansprucht, dadurch gekennzeichnet, daß ein Ausgangsmaterial verwendet wird, worin A 2-Benzothiazolyl ist.

12. Verfahren, wie in einem der Ansprüche 1 bis 5 beansprucht, dadurch gekennzeichnet, daß ein Ausgangsmaterial verwendet wird, worin A 2-Naphthalenyl oder 2-Chinolinyl ist.

13. Verfahren, wie in einem der Ansprüche 1 bis 5 beansprucht, dadurch gekennzeichnet, daß ein Ausgangsmaterial verwendet wird, worin A 2-Phenyl-4-thiazolyl ist.

14. Verfahren, wie in einem der Ansprüche 1 bis 5 beansprucht, dadurch gekennzeichnet, daß ein Ausgangsmaterial verwendet wird, worin A 2,2-Dichlorvinyl oder 2,2-Dimethylvinyl ist.

15. Verfahren zum Herstellen eines pharmazeutisch annehmbaren Salzes einer Verbindung der Formel (I), wie in Anspruch 1 definiert, welches Verfahren das Behandeln einer Verbindung der Formel (I), die zumindest ein Stickstoffatom enthält, mit einer pharmazeutisch annehmbaren Säure zur Bildung eines Säureadditionssalzes oder das Behandeln einer Verbindung der Formel (I), wie in Anspruch 1 definiert, mit einer Base eines pharmazeutisch annehmbaren Kations zur Bildung eines Salzes der Carbonsäurefunktion der Verbindung der Formel (I) umfaßt.

16. Verfahren, wie in Anspruch 15 beansprucht, dadurch gekennzeichnet, daß eine Verbindung der Formel (I), wie in Anspruch 1 definiert, mit einer Alkalimetallbase zur Bildung eines Alkalimetallsalzes der Carbonsäurefunktion umgesetzt wird.

17. Verfahren, wie in Anspruch 15 beansprucht, dadurch gekennzeichnet, daß eine Verbindung der Formel (I), wie in Anspruch 1 definiert, mit einem Amin zur Bildung eines Aminsalzes der Carbonsäurefunktion umgesetzt wird.

18. Verfahren zum Herstellen einer pharmazeutischen Zusammensetzung, dadurch gekennzeichnet, daß eine Verbindung der Formel (I), wie in Anspruch 1 definiert, oder ein pharmazeutisch annehmbares Salz hievon mit einem pharmazeutischen Träger gemischt und die erhaltene Zusammensetzung in eine für therapeutische Verabreichung geeignete Form gebracht wird.

19. Verfahren, wie in Anspruch 18 beansprucht, dadurch gekennzeichnet, daß die Verbindung der Formel (I) durch ein Verfahren, wie in einem der Ansprüche 1 bis 14 beansprucht, hergestellt wird.

20. Verwendung einer Verbindung der Formel (I), wie in Anspruch 1 definiert, oder eines pharmazeutisch annehmbaren Salzes hievon zur Herstellung eines Medikaments zur Verwendung als entzündungshemmendes, antiallergisches oder zellenschützendes Mittel.

21. Verfahren zum Herstellen eines Ätheresters der Formel (IV), wie in Anspruch 1 definiert, dadurch gekennzeichnet, daß eine Verbindung der Formel (II)
$ACH_2$-Hal (II),
worin A wie in Anspruch 1 definiert ist und Hal ein Halogenatom ausgewählt aus Chlor, Brom und Jod bedeutet, mit einem Dimetallderivat einer Verbindung der Formel (III)

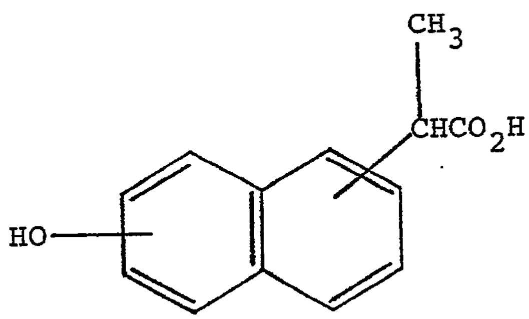
(III)

umgesetzt wird.

22. Verfahren, wie in Anspruch 21 beansprucht, dadurch gekennzeichnet, daß das Metallderivat der Verbindung der Formel (III) ein Dialkalimetallderivat ist.

**Revendications pour les Etats Contractants: AT, BE, CH, DE, FR, IT, LI, LU, NL, SE**

1. Composé de formule:

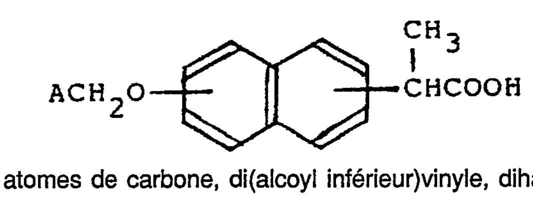

où A est alcoyle de 3 à 19 atomes de carbone, di(alcoyl inférieur)vinyle, dihalovinyle, diphénylvinyle, alcynyle inférieur,

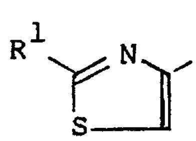
ou
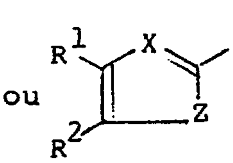
;

X est N ou CR;
Z est -CR=CR-, -CR=N-, -N=CR-, -NR-, S ou O;

R est hydrogène ou alcoyle inférieur;
$R^1$ est hydrogène, alcoyle inférieur ou phényle;
$R^2$ est hydrogène ou alcoyle inférieur; ou
$R^1$ et $R^2$ pris ensemble avec les atomes de carbone auxquels ils sont unis forment un cycle benzénique;
ou un sel pharmaceutiquement acceptable de celui-ci, avec la restriction que A ne compte pas 3 à 5 atomes de carbone lorsque $ACH_2O$ occupe la position 6.

2. Composé de formule I suivant la revendication 1, dans lequel A est

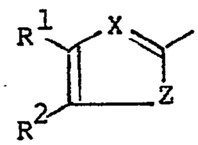 ,

X est CR, Z est -N=CR- et $R^1$ et $R^2$ pris ensemble avec les atomes de carbone auxquels ils sont unis forment un cycle benzénique.

3. Composé de formule I suivant la revendication 1, dans lequel A est

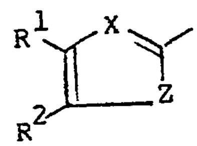 ,

X est CR, Z est -CR=CR- et $R^1$ et $R^2$ sont hydrogène.

4. Composé de formule I suivant la revendication 1, dans lequel A est

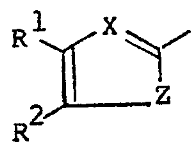 ,

X est N, Z est -NR- et $R^1$ et $R^2$ pris ensemble avec les atomes de carbone auxquels ils sont unis forment un cycle benzénique.

5. Composé de formule I suivant la revendication 1, dans lequel A est

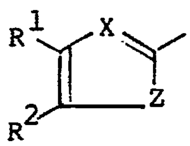 ,

X est N, Z est -N=CR- et $R^1$ et $R^2$ sont hydrogène.

6. L'acide α-méthyl-6-(2-quinolinylméthoxy)-2-naphtalène-acétique ou un sel pharmaceutiquement acceptable de celui-ci.

7. L'acide α-méthyl-6-(phénylméthoxy)-2-naphtalène-acétique ou un sel pharmamceutiquement acceptable de celui-ci.

8. L'acide α-méthyl-6-[(1-méthyl-1H-benzimidazol-2-yl)méthoxy]-2-naphtalène-acétique ou un sel pharmaceutiquement acceptable de celui-ci.

9. L'acide α-méthyl-6-(2-pyridinylméthoxy)-2-naphtalène-acétique ou un sel pharmaceutiquement acceptable de celui-ci.

10. L'acide α-méthyl-6-(2-benzothiazolylméthoxy)-2-naphtalène-acétique ou un sel pharmaceutiquement acceptable de celui-ci.

11. L'acide α-méthyl-6-(2-naphtalénylméthoxy)-2-naphtalène-acétique ou un sel pharmaceutiquement acceptable de celui-ci.

12. L'acide S-(+)-α-méthyl-6-(2-quinolinylméthoxy)-2-naphtalène-acétique, l'acide R-(−)-α-méthyl-6-(2-quinolinylméthoxy)-2-naphtalène-acétique ou un sel pharmaceutiquement acceptable de ceux-ci.

13. L'acide α-méthyl-6-[(2-phényl-4-thiazolyl)méthoxy]-2-naphtalène-acétique ou un sel pharmaceutiquement acceptable de celui-ci.

14. L'acide α-méthyl-6-(dodécyloxy)-2-naphtalène-acétique, l'acide α-méthyl-6-(3-méthyl-2-buténoxy)-2-naphtalène-acétique ou l'acide α-méthyl-6-(3,3-dichloroallyloxy)-2-naphtalène-acétique ou un sel pharmaceutiquement acceptable de ceux-ci.

15. Sel de métal alcalin d'un composé suivant l'une quelconque des revendications précédentes.

16. Sel d'amine d'un composé suivant l'une quelconque des revendications 1 à 14.

17. Le sel de sodium de l'acide α-méthyl-6-(2 quinolinylméthoxy)-2-naphtalène-acétique.

18. Composition pharmaceutique comprenant un composé suivant l'une quelconque des revendications 1 à 17, et un excipient pharmaceutiquement acceptable.

19. Composé suivant l'une quelconque des revendications 1 à 17, à utiliser comme agent antiinflammatoire, anti-allergique ou cytoprotecteur.

20. Procédé de préparation d'un composé de formule I suivant la revendication 1 ou d'un sel pharmaceutiquement acceptable de celui-ci, caractérisé en ce qu'un éther-ester de formule IVa

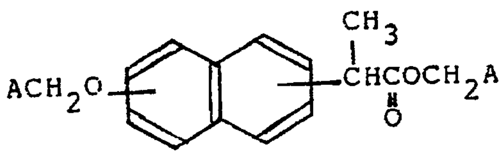

où A est tel que défini dans la revendication 1 est hydrolysé et, si la chose est souhaitée, le composé de formule I est isolé à l'état de sel pharmaceutiquement acceptable.

21. Procédé de préparation d'un composé de formule I tel que défini dans la revendication 1 ou d'un sel pharmaceutiquement acceptable de celui-ci, caractérisé en ce qu'un composé de formule II

$ACH_2Hal$ II

où A est tel que défini dans la revendication 1 et Hal est un atome d'halogène choisi entre le chlore, le brome et l'iode, est mis à réagir avec un dérivé dimétallique d'un composé de formule III

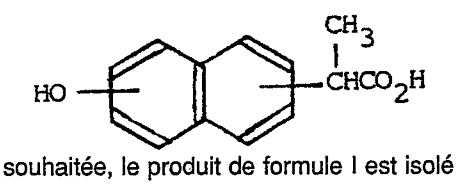

III

et, si la chose est souhaitée, le produit de formule I est isolé à l'état de sel pharmaceutiquement acceptable.

22. Composé de formule IVa ou un sel pharmaceutiquement acceptable de celui-ci

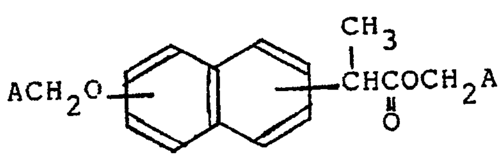

où A est tel que défini dans la revendication 1.

23. Composé de formule IV ou un sel pharmaceutiquement acceptable de celui-ci

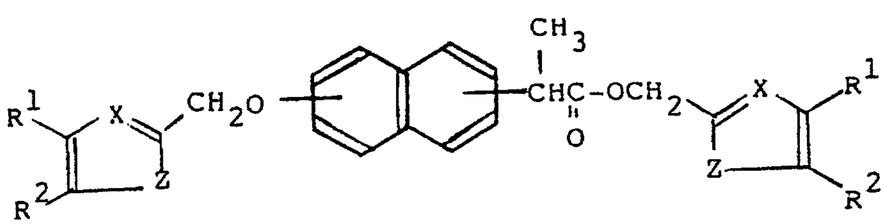

où $R^1$, $R^2$, X et Z sont tels que définis dans la revendication 1.

24. Procédé de préparation d'un composé de formule I suivant la revendication 1 ou d'un sel pharmaceutiquement acceptable de celui-ci, caractérisé en ce qu'un ester de formule IVb

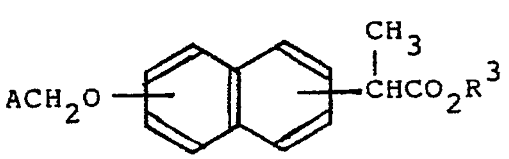

où A est tel que défini dans la revendication 1 et $R^3$ est un radical alcoyle de 1 à 6 atomes de carbone, est hydrolysé et, si la chose est souhaitée, le produit de formule I est isolé à l'état de sel pharmaceutiquement acceptable.

**Revendications pour les Etats Contractants: ES, GR**

1. Procédé de préparation d'un composé de formule:

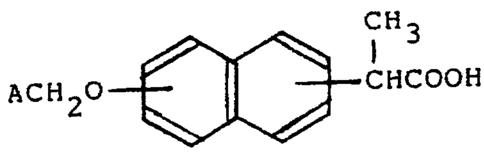

où A est alcoyle de 3 à 19 atomes de carbone, di(alcoyl inférieur)vinyle, dihalovinyle, diphénylvinyle, alcynyle inférieur,

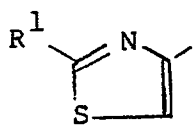

ou

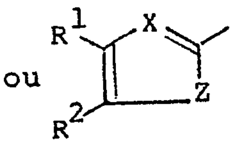

;

X est N ou CR;
Z est -CR=CR-, -CR=N-, -N=CR-, -NR-, S ou O;
R est hydrogène ou alcoyle inférieur;
$R^1$ est hydrogène, alcoyle inférieur ou phényle;
$R^2$ est hydrogène ou alcoyle inférieur; ou
$R^1$ et $R^2$ pris ensemble avec les atomes de carbone auxquels ils sont unis forment un cycle benzénique;
ou d'un sel pharmaceutiquement acceptable de celui-ci, caractérisé en ce qu'un éther-ester de formule IVa

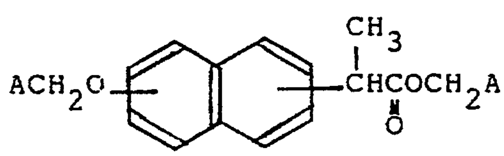

où A est tel que défini ci-dessus, est hydrolysé pour donner un composé de formule I qui, si la chose est souhaitée, est isolé à l'état de sel pharmaceutiquement acceptable, avec la restriction que A ne compte pas 3 à 5 atomes de carbone lorsque $ACH_2O$ occupe la position 6.

2. Procédé suivant la revendication 1, caractérisé en ce que le composé de départ de formule IV est préparé par réaction d'un composé de formule II:

$ACH_2Hal$ II

où A est tel que défini ci-dessus et Hal est un atome d'halogène choisi entre le chlore, le brome et l'iode, avec un dérivé métallique d'un composé de formule III:

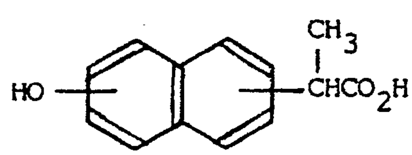

III

3. Procédé de préparation d'un composé de formule I tel que défini dans la revendication 1, caractérisé en ce qu'un composé de formule II:

$ACH_2Hal$ II

où A est tel que défini ci-dessus et Hal est un atome d'halogène choisi entre le chlore, le brome et l'iode, est mis à réagir avec un dérivé dimétallique d'un composé de formule III:

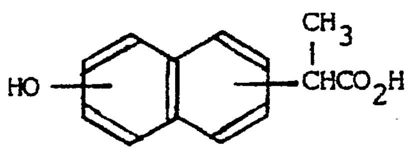

III

et si la chose est souhaitée, le produit est isolé à l'état de sel pharmaceutiquement acceptable.

4. Procédé suivant la revendication 2 ou 3, caractérisé en ce que le dérivé métallique du composé de formule III est préparé par traitement du composé de formule III à l'aide d'un alcoolate de métal alcalin.

5. Procédé de préparation d'un composé de formule I tel que défini dans la revendication 1 ou d'un sel pharmaceutiquement acceptable de celui-ci, caractérisé en ce qu'un ester de formule IVb:

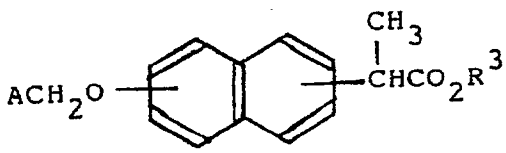

où A est tel que défini dans la revendication 1 et $R^3$ est un radical alcoyle de 1 à 6 atomes de carbone, est hydrolysé et si la chose est souhaitée, le produit de formule I est isolé à l'état de sel pharmaceutiquement acceptable.

6. Procédé suivant l'une quelconque des revendications 1 à 5, caractérisé en ce qu'on utilise un composé de départ dans lequel A est

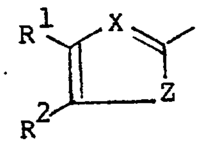

,

X est CR, Z est -N=CR- et $R^1$ et $R^2$ pris ensemble avec les atomes de carbone auxquels ils sont unis forment un cycle benzénique.

7. Procédé suivant l'une quelconque des revendications 1 à 5, caractérisé en ce qu'on utilise un composé de départ dans lequel A est

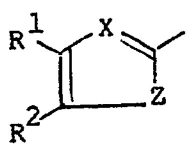

,

X est CR, Z est -CR=CR- et $R^1$ et $R^2$ sont hydrogène.

8. Procédé suivant l'une quelconque des revendications 1 à 5, caractérisé en ce qu'on utilise un composé de départ dans lequel A est

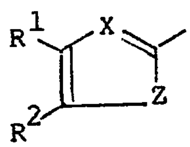

,

X est N, Z est -NR- et $R^1$ et $R^2$ pris ensemble avec les atomes de carbone auxquels ils sont unis forment un cycle benzénique.

9. Procédé suivant l'une quelconque des revendications 1 à 5, caractérisé en ce qu'on utilise un composé de départ dans lequel A est

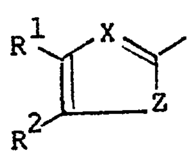

,

X est N, Z est -N=CR- et $R^1$ et $R^2$ sont hydrogène.

10. Procédé suivant l'une quelconque des revendications 1 à 5, caractérisé en ce qu'on utilise un composé de départ dans lequel A est alcoyle de 3 à 19 atomes de carbone.

11. Procédé suivant l'une quelconque des revendications 1 à 5, caractérisé en ce qu'on utilise un composé de départ dans lequel A est benzothiazolyle.

12. Procédé suivant l'une quelconque des revendications 1 à 5, caractérisé en ce qu'on utilise un composé de départ dans lequel A est 2-naphtalényle ou quinolinyle.

13. Procédé suivant l'une quelconque des revendications 1 à 5, caractérisé en ce qu'on utilise un composé de départ dans lequel A est 2-phényl-4-thiazolyle.

14. Procédé suivant l'une quelconque des revendications 1 à 5, caractérisé en ce qu'on utilise un composé de départ dans lequel A est 2,2-dichlorovinyle ou 2,2-diméthylvinyle.

15. Procédé de préparation d'un sel pharmaceutiquement acceptable d'un composé de formule I tel que défini dans la revendication 1, lequel procédé comprend le traitement d'un composé de formule I contenant au moins un atome d'azote à l'aide d'un acide pharmaceutiquement acceptable pour former un sel d'addition d'acide ou le traitement d'un composé de formule I tel que défini dans la revendication 1 à l'aide d'une base d'un cation pharmaceutiquement acceptable pour former un sel de la fonction acide carboxylique du composé de formule I.

16. Procédé suivant la revendication 15, caractérisé en ce qu'un composé de formule I tel que défini dans la revendication 1 est mis à réagir avec une base de métal alcalin pour former un sel de métal alcalin de la fonction acide carboxylique.

17. Procédé suivant la revendication 15, caractérisé en ce qu'un composé de formule I tel que défini dans la revendication 1 est mis à réagir avec une amine pour former un sel d'amine de la fonction acide carboxylique.

18. Procédé de préparation d'une composition pharmaceutique, caractérisé en ce qu'un composé de formule I tel que défini dans la revendication 1, ou un sel pharmaceutiquement acceptable de celui-ci, est mélangé avec un excipient pharmaceutiquement acceptable et la composition résultante est présentée sous une forme propre à l'administration thérapeutique.

19. Procédé suivant la revendication 18, caractérisé en ce que le composé de formule I est préparé par un procédé suivant l'une quelconque des revendications 1 à 14.

20. Utilisation d'un composé de formule I tel que défini dans la revendication 1 ou d'un sel pharmaceutiquement acceptable de celui-ci pour la production d'un médicament à utiliser comme agent anti-inflammatoire, antiallergique ou cytoprotecteur.

21. Procédé de préparation d'un éther-ester de formule IV tel que défini dans la revendication 1, caractérisé en ce qu'un composé de formule II

$ACH_2Hal$ II

où A est tel que défini dans la revendication 1 et Hal est un atome d'halogène choisi entre le chlore, le brome et l'iode, est mis à réagir avec un dérivé dimétallique d'un composé de formule III:

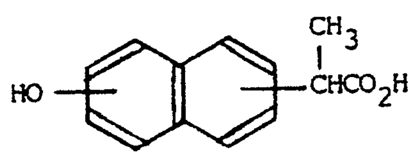

III

22. Procédé suivant la revendication 21, caractérisé en ce que le dérivé métallique du composé de formule III est un dérivé de dimétal alcalin.